(19)

**Europäisches Patentamt**

**European Patent Office**

**Office européen des brevets**

(11) **EP 4 659 796 A2**

(12) **EUROPEAN PATENT APPLICATION**

(43) Date of publication:
**10.12.2025 Bulletin 2025/50**

(21) Application number: **25197469.7**

(22) Date of filing: **08.07.2020**

(51) International Patent Classification (IPC):
***A61N 1/26*** (2006.01)

(52) Cooperative Patent Classification (CPC):
**A61N 1/3787;** A61N 1/3605; A61N 1/37217;
A61N 1/375

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.07.2019 US 201916504623
14.02.2020 US 202062976698 P**

(62) Document number(s) of the earlier application(s) in
accordance with Art. 76 EPC:
**20837675.6 / 3 996 798**

(71) Applicant: **Bioness Inc.
Valencia, CA 91355 (US)**

(72) Inventors:
• **GLUKHOVSKY, Arkady
Valencia, CA, 91355 (US)**

• **MCBRIDE, Keith
Ventura, CA, 93003 (US)**

(74) Representative: **Gill Jennings & Every LLP
The Broadgate Tower
20 Primrose Street
London EC2A 2ES (GB)**

Remarks:
•This application was filed on 21/08/2025 as a
divisional application to the application mentioned
under INID code 62.
•Claims filed after the date of filing of the application/
the date of receipt of the divisional application (Rule
68(4) EPC).

(54) **APPARATUS AND METHODS FOR PROVIDING ELECTRIC ENERGY TO A SUBJECT**

(57)     An apparatus includes a power adapter having a housing and a circuit at least partially disposed in the housing. The housing is configured to be coupled to an implantable device for disposition in a body. The circuit is configured to be electrically connected to a power circuit of the implantable device when the housing is coupled to the implantable electrical conductor. When the housing is coupled to the implantable electrical conductor and implanted in a body, the circuit is configured to (1) receive, transcutaneously from a power supply, a first energy, (2) convert the first energy to a second energy, and (3) transfer, to the implantable device, the second energy such that the second energy powers the implantable device.

FIG. 1A

EP 4 659 796 A2

**Description**

**CROSS-REFERENCE TO RELATED APPLICATIONS**

This application claims priority to and the benefit of U.S. Patent Application Serial No. 16/504,623 filed July 8, 2019, entitled, "Implantable Power Adapter," the disclosure of which is incorporated herein by reference in its entirety.
This application also claims priority to and the benefit of U.S. Provisional Patent Application Serial No. 62/976,698 filed February 14, 2020, entitled, "Apparatus and Methods for Providing Electric Energy to a Subject," the disclosure of which is incorporated herein by reference in its entirety.

**BACKGROUND**

The present disclosure relates generally to the field of electrical transmitters or stimulators used alone or in conjunction with implantable electrical devices, and in particular, to external electrical transmitters configured to provide transcutaneous electrical energy to a portion of a subject and/or to an implant disposed in the subject, and power adapters that are configured to be used with such an implant.
Providing basic transcutaneous electrical nerve stimulation to a subject's body is known. In general, an external transmitter or stimulator (or electrical contacts thereof) can be placed in contact with the subject's skin and can provide stimulation directly to a target area or nerve or can provide electrical energy to an implanted device, which in turn, provides stimulation to the target area or nerve. In some known implementations, implantable devices receive power and/or energy from an external transmitter that can transcutaneously apply and/or provide a low frequency electrical current, which is received and/or "picked-up" by one or more electrodes or conductive portions of the implantable device. In this manner, the energy transfer can be similar to the transcutaneous energy transfer used in some known devices for delivering direct transcutaneous electrical nerve stimulation.
Providing low frequency electrical current to a target area of the subject and/or to implantable devices, however, can cause pain, muscle contraction and/or activation, discomfort, and/or other undesirable sensations at or in non-target areas of the subject. Sensitivity (e.g., of a body) to a transcutaneous electrical stimulus decreases as the frequency of the stimulus increases, which can lower undesirable sensitivity but can also lower efficacy of the implantable device if the device, in turn, transmits electrical stimulus with undesirable characteristics (e.g., relatively high frequencies). In addition to sensitivity, the use of an external transmitter or stimulator to provide transcutaneous electrical stimulus to the target area of the subject (e.g., either directly or via an implantable device) can result in electrical charge build up on the skin and/or can result in undesirable heating of the external transmitter or stimulator associated with, for example, switching between positive and negative phases of the stimulation.
Thus, a need exists for external transmitters or stimulators that can provide transcutaneous energy to a target area of a subject and/or to implantable devices implanted in the subject while limiting undesirable interactions with other or non-target portions of the body. Furthermore, a need exists for implantable devices and/or power adapters used with implantable devices that receive transcutaneous energy having a desired set of characteristics to avoid causing pain, muscle contractions and/or activation, discomfort, and other undesirable sensations to portions of a subject (e.g., non-target areas or tissue).

**SUMMARY**

In some embodiments, an apparatus includes a housing and a circuit at least partially disposed in the housing. The housing can be configured to be coupled to an implantable electrical conductor for disposition in a body. The circuit can be configured to be electrically connected to a pick-up electrode of the implantable electrical conductor when the housing is coupled to the implantable electrical conductor. When the housing is coupled to the implantable electrical conductor and implanted in a body, the circuit is configured to (1) receive, transcutaneously from a power supply, a first energy, (2) convert the first energy to a second energy, and (3) transmit, to the pick-up electrode, the second energy such that the implantable electrical conductor can apply, via a stimulating electrode, the second energy at the second frequency to a region in the body.

**BRIEF DESCRIPTION OF THE DRAWINGS**

Unless otherwise indicated, the drawings are not necessarily to scale. The drawings are merely schematic representations, not necessarily intended to portray specific parameters of the invention. The drawings are intended to depict only typical embodiments of disclosed systems, apparatus, and methods.

FIG. 1A is a schematic block diagram depicting a power adapter coupled to an implant, in accordance with an embodiment.

FIG. 1B is a schematic block diagram depicting an implant without a power adapter, in accordance with an embodiment.

FIG. 2 is a schematic block diagram depicting a power adapter, in accordance with an embodiment.

FIG. 3 is a schematic block diagram depicting an example of use of an apparatus in conjunction with a transmitter, in accordance with an embodiment.

FIG. 4 is a flowchart depicting a method of using a power adapter, in accordance with an embodiment.

FIGS. 5A and 5B are schematic diagrams depicting an effect of using a power adapter in conjunction with a transmitter, in accordance with an embodiment.

FIGS. 5C-5E are waveforms illustrating potential waveforms used with respect to a power adapter, in accordance with an embodiment.

FIG. 5F is a graph illustrating the relationship between charge and frequency when applied to an individual, according to an embodiment.

FIGS. 6A-6F depict various views of a power adapter and/or an implant, in accordance with an embodiment.

FIGS. 7A and 7B depict a side view and a partial cross-sectional perspective view, respectively, of a power adapter and an implant, in accordance with an embodiment.

FIGS. 8A-8C are schematic diagrams depicting circuits of a power adapter, in accordance with various embodiments.

FIGS. 9A and 9B are schematic diagrams depicting circuits of a power adapter, in accordance with various embodiments.

FIGS. 10A and 10B are schematic diagrams depicting circuits of a power adapter, in accordance with various embodiments.

FIG. 11A depicts a non-rectified waveform (e.g., an alternating current waveform), in accordance with an embodiment.

FIG. 11B depicts a one-way rectified waveform, in accordance with an embodiment.

FIG. 11C depicts a two-way rectified waveform, in accordance with an embodiment.

FIGS. 12A-12D are schematic diagrams depicting at least a portion of a power adapter, in accordance with various embodiments.

FIG. 13 depicts a transmitter, and a power adapter coupled to an implant, in accordance with an embodiment.

FIG. 14A is a schematic diagram depicting a kit including an implantable device and associated implements, in accordance with an embodiment.

FIG. 14B is a schematic diagram depicting a kit including implements for coupling a power adapter to an implant, in accordance with an embodiment.

FIGS. 15A and 15B are schematic diagrams depicting at least a portion of an external pulse transmitter electrically connected to a load, in accordance with an embodiment.

FIGS. 16A-16D are graphs illustrating various relationships between compliance voltage and load voltage, in accordance with an embodiment.

FIGS. 17A-17D are schematic diagrams depicting a portion of a circuit included in the external pulse transmitter of FIGS. 15A and 15B, and shown in a first configuration, a second configuration, a third configuration, and a fourth configuration, respectively.

FIG. 17E is a graph illustrating a current and a voltage across a load as the portion of the circuit of FIGS. 17A-17D is transitioned between the first configuration, the second configuration, the third configuration, and the fourth configuration.

FIG. 18 depicts a waveform generated by an external pulse transmitter, in accordance with an embodiment.

FIGS. 19A and 19B are graphs of an oscilloscope depicting a first waveform and a second waveform, respectively, each of which is generated by an external pulse transmitter, in accordance with an embodiment.

FIG. 20 is a schematic diagram depicting at least a portion of a power adapter, in accordance with an embodiment.

FIG. 21 is a schematic diagram depicting at least a portion of a power adapter, in accordance with an embodiment.

## DETAILED DESCRIPTION

The embodiments and/or methods described herein are related to external electrical transmitters or stimulators configured to provide transcutaneous electrical energy to a target area of a subject and/or to an implantable device implanted in the subject, and power adapters configured for use with at least some implantable devices. In some embodiments, for example, a method of using an external electrical transmitter to transcutaneously provide electrical energy to an implant implanted in a subject includes providing a first electrical pulse while a stimulating circuit is in a first configuration in which a power source (e.g., a voltage source and/or a current source) is electrically connected to a load. The first electrical pulse having a positive voltage. The load can be, for example, a portion of the skin of the subject positioned between the external electrical transmitter and the implant, which can have a capacitive element (or property) that results in electrical charge build up across the load. The stimulating circuit is transitioned to a second configuration in which the power source is electrically isolated from the load to allow the load to discharge the electrical charge built up across the load. The stimulating circuit is transitioned, after a predetermined time, to a third configuration different from the first configuration and the second configuration, in which the power source is electrically connected to the load. The method includes providing a second electrical pulse while the stimulating circuit is in the third configuration. The second electrical pulse having a negative voltage (or vice versa).

In some embodiments, an apparatus includes a housing and a circuit at least partially disposed in the housing (e.g., as part of a power adapter). The housing can be configured to be coupled to an implantable electrical conductor for disposition in a body. The circuit can be configured to be electrically connected to a pick-up electrode of the implantable electrical conductor when the housing is coupled to the implantable electrical conductor. When the housing is coupled to the implantable electrical conductor and implanted in a body, the circuit is configured to (1) receive, transcutaneously from a power supply (e.g., an external electrical transmitter), a first energy, (2) convert the first energy to a second energy, and (3) transmit, to the pick-up electrode, the second energy such that the implantable electrical conductor can apply, via a stimulating electrode, the second energy at the second frequency to a region in the body.

In some embodiments, an apparatus includes a power adapter having a housing and a circuit at least partially disposed in the housing. The housing can be configured to be coupled to an implantable device for disposition in a body. The circuit can be configured to be electrically connected to the implantable device when the housing is coupled to the implantable electrical conductor. When the housing is coupled to the implantable electrical conductor and implanted in a body, the circuit can be configured to (1) receive, transcutaneously from a power supply (e.g., an external electrical transmitter), a first energy having a first set of characteristics, (2) convert the first energy to a second energy having a second set of characteristics different from the first set of characteristics, and (3) transfer, to the implantable device, the second energy such that the second energy powers the implantable device.

In some embodiments, a method includes receiving, transcutaneously and from an external electrical transmitter, first energy having a first set of characteristics. The first energy is converted, via a rectification circuit, to a second energy having a second set of characteristics different from the first set of characteristics. The second energy is transferred from the rectification circuit to a stimulating electrode of an implantable electrical conductor such that the implantable electrical conductor applies, via the stimulating electrode, the second energy to a target nerve internal to a body.

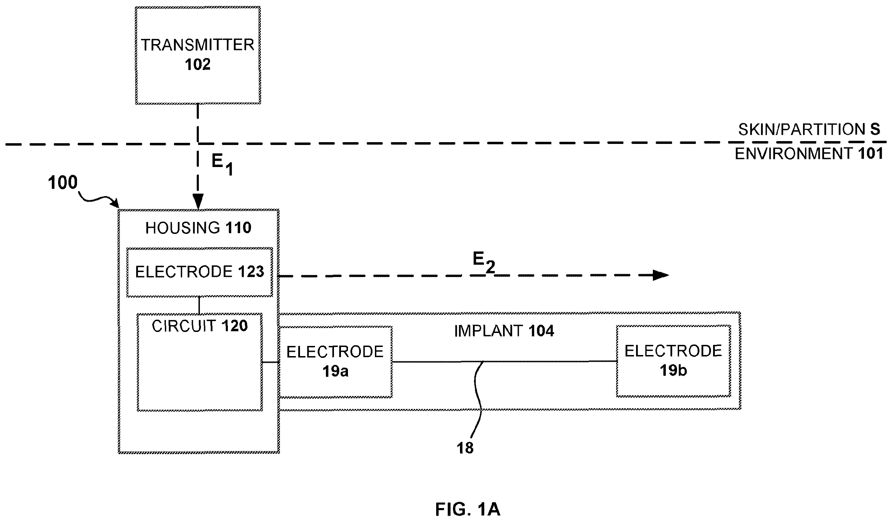

FIG. 1A is a schematic block diagram depicting a power adapter 100 coupled to an implant 104, in accordance with an embodiment. As shown in FIG. 1A, the power adapter 100 includes a housing 110, a circuit 120 at least partially disposed in the housing 110 and an electrode 123. The power adapter 100 can be configured to be coupled or interconnected to implant 104 such as at and via the housing 110, as shown in FIG. 1A. The power adapter 100 can be configured to operate, in conjunction with and when coupled to the implant 104, in an environment of and internal to a body, such as environment 101, which can be defined, for example, by a boundary such as skin/partition S, such as shown in FIG. 1A.

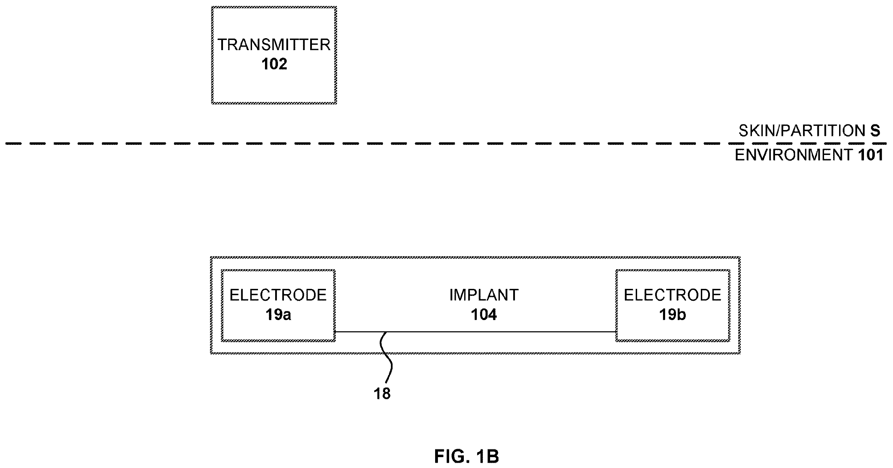

FIG. 1B is a schematic block diagram depicting the implant 104 without a power adapter being couple thereto (e.g., the power adapter 100 shown in FIG. 1A). As shown in FIG. 1B, the implant 104 includes electrodes 19a and 19b. The power adapter 100, when coupled to the implant 104 (e.g., at and/or over electrode 19a such as shown in FIG. 1A), can be configured to operate in the environment 101, in conjunction with the implant 104 and a device such as an external electrical transmitter 102 to, for example, enable (e.g., supply power to) the implant 104 to perform or otherwise carry out a medical procedure, task, operation, or measurement in the body. More specifically, the electrode 123 can be configured to receive electrical energy from external electrical transmitter 102, the circuit 120 can convert the frequency and/or waveform of the electrical energy, and the power adapter 100 can provide the converted electrical energy to the electrode 19a, as described in further detail herein.

For example, the power adapter 100 can be configured to receive, from the external electrical transmitter 102 and via the electrode 123, energy $E_1$ (referred to herein as "first energy") such as a first form or quantity of energy, power, or signals (collectively, "energy") having a first characteristic or set of characteristics (e.g., a first frequency, a first waveform, a first burst pattern, and/or the like). The first energy $E_1$, due to the first characteristic(s), may be unsuitable for use in powering and/or otherwise provided to or used by the implant 104. Accordingly, to provide energy suitable for use by the implant 104 such that the implant 104 is enabled to perform the medical procedure in the body, the power adapter 100 can be configured to transform, rectify, derive, adapt, and/or otherwise convert the first energy $E_1$ to a second energy $E_2$, including a second form or quantity of energy, power, or signals ("collectively, energy") having a second characteristic or set of characteristics (e.g., a second frequency, a second waveform, a second burst pattern, and/or the like). As shown in FIG. 1A, the power adapter 100 can be configured to convert the first energy $E_1$ to the second energy $E_2$ such that the second energy $E_2$, due to the second characteristic(s), is suitable for use by the implant 104, such that the implant 104 is enabled to perform, using the second energy $E_2$, the medical procedure in the body (e.g., including providing, via the second energy $E_2$, stimulation, activation, or excitation of tissue, nerves, or muscles in the body, or blocking of excitation or activation of the tissue, nerves, or muscles). The power adapter 100 can be configured to transfer or input the second energy $E_2$ to the implant 104 to enable (e.g., powering of, or control over) the implant 104 in performing or otherwise carrying out the medical procedure in the body. More particularly, when the power adapter 100 is coupled to the implant 104, the circuit 120 is electrically coupled to the electrode 19a (e.g., via a conductor or the like not shown in FIG. 1A) such that the second energy $E_2$ that is generated by the circuit 120 is provided as an input to the electrode 19a.

The housing 110 can be configured to be coupled to the implant 104 for disposition in a body therewith. The electrode 123 of the power adapter 100 can be configured to receive, transcutaneously with respect to the body, the first energy $E_1$ (e.g., high frequency electrical bursts, low frequency pulses, etc.) for conversion and transfer to implant 104 for application (e.g., in the form of bursts or pulses, to be used by the implant 104, etc.), as described herein. Skin/partition S can include, for example, a barrier, partition, skin, and the like, such as of the body of a subject, including, for example, a person, patient, and the like. The body of the subject can include an (e.g., internal) environment, such as environment 101.

The external electrical transmitter 102 can be or include, for example, an external pulse transmitter (EPT), a power source or supply, an energy source or supply, a voltage source or supply, a (wireless) energy transfer device, a signal transmitter, and/or the like. The external electrical transmitter 102 can be any suitable configuration. Various terms are used herein to refer to an external transmitter and should be understood to be interchangeable unless the context clearly dictates otherwise. For example, such terms can include "external electrical transmitter," "external electrical stimulator," "external pulse transmitter," "external pulse generator," "electrical pulse generator," "electrical transmitter," "electrical stimulator," "power supply," "transcutaneous electrical nerve stimulator (TENS)," and/or the like. For simplicity, the term "transmitter" is generally used herein to refer to such devices.

The transmitter 102 can be configured to transmit energy (e.g., the first energy $E_1$) into a body of a subject, which can be received, for example, by the power adapter 100 and used in and/or by implant 104 (e.g., when power adapter 100 is coupled to implant 104). For example, the transmitter 102 can be configured to transmit the energy into the body for receipt, or pick-up (e.g., of some portion of the energy), by the power adapter 100. Subsequently, the energy, after being received by the power adapter 100, can be transferred from the power adapter 100 to the implant 104 (e.g., the second energy $E_2$, shown in FIG. 1A). In some instances, the energy can be converted to a form (e.g., from a first form of energy to a second form of energy) suitable for use in powering the implant 104, such as to enable the implant 104 to perform a medical procedure in the body, as described herein. In other instances, the power adapter 100 can have and/or can be placed in a pass-through configuration and/or state in which the energy received from the transmitter is transferred to the implant without substantially modifying the characteristics of the energy. Accordingly, the second energy $E_2$ transferred from the power adapter 100 to the implant 104 can have characteristics similar to or different from characteristics of the first energy $E_1$ received from the transmitter 102.

The transmitter 102 can be configured to transmit the energy into a body of a subject transcutaneously, at various levels of current, or electrical charge, and at current and/or frequency levels, to avoid causing adverse sensory or motor activation or stimulation (e.g., responses at or in undesirable locations of the body near the electrodes of the transmitter 102, referred to herein as a "local response") in and by the body. In some instances, the transmitter 102 can deliver energy transcutaneously via hydrogel, wetted cloth, and/or other electrodes attached to the skin. In some instances, the transmitter 102 can be configured to transmit the energy via output of a time-varying voltage (or electrical potential), current (or electrical charge), or electromagnetic field - at a predetermined frequency or range of frequencies, and with a predetermined waveform. In some implementations, the output from the transmitter 102 can include, for example, a time-varying flow of electrical charge. The time-varying flow of electrical charge can include, for example, electrical bursts, electrical pulses, and/or the like ("electrical burst(s)" or "burst(s)"), such as in the form of a train or series of high frequency bursts, including, for example, electrical, electromagnetic, and/or magnetic bursts. In some implementations, the output of the transmitter 102 can include a train or series of low frequency bursts, where each burst includes a single low frequency pulse. In some implementations, the output of the transmitter 102 can include a train or series of bursts including any suitable combination of one or more low frequency energy bursts and one or more high frequency energy bursts. In some instances, the one or more low frequency energy bursts can have one or more characteristics configured to result in a desirable local response in and by the body such as, for example, increased blood flow within a region of the body adjacent to or relatively near the transmitter 102, while the one or more high frequency energy bursts can be received by, for example, the power adapter 100.

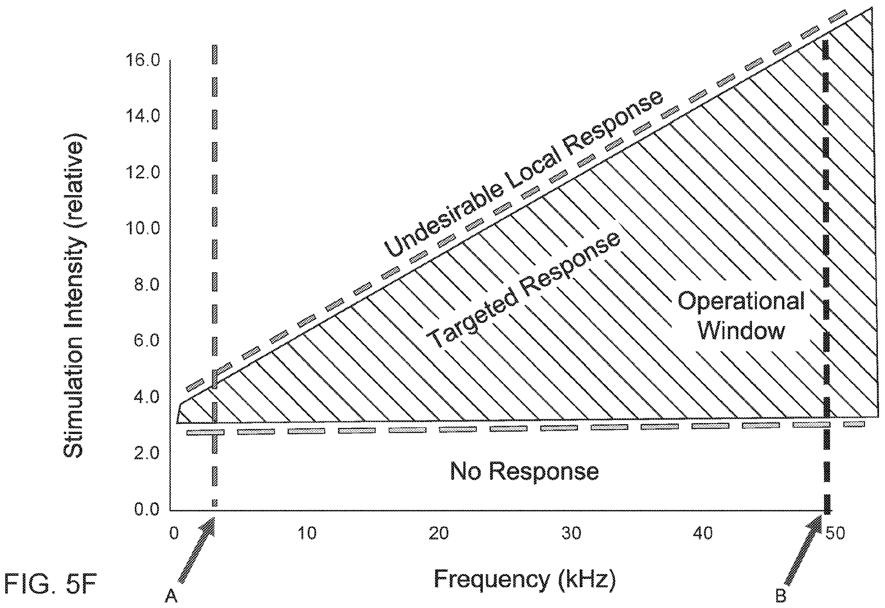

In some implementations, the predetermined frequency or range of frequencies can include, for example, a frequency or range of frequencies between approximately 10 kilohertz (kHz) and 100 kHz. The predetermined frequency or range of frequencies can otherwise include a frequency or range of frequencies at which the energy output from the transmitter 102 can be applied, such as to a body of a subject, without causing an undesirable response, or stimulation ("response"), such as an undesirable local motor response, in and by the body near the electrodes of the transmitter. For example, FIG. 5F is a graph illustrating a relative stimulation intensity as a function of stimulation frequency. In some implementations, the transmitter 102 can be configured to provide energy or stimulation at a frequency within the shaded area identified as the "Operational Window." Moreover, as the frequency increases, the stimulation intensity prior to causing an undesirable local response also increases, as described in further detail herein.

In some implementations, the transmitter 102 can be configured to transmit the energy (e.g., first energy $E_1$) at a frequency and charge configured to avoid causing a sensation or response in or by the body tissues (e.g., a local response). In some implementations, the transmitter 102 can be configured to transmit energy (e.g., the first energy $E_1$) at a frequency and charge configured to cause a desirable local response (e.g., increased blood flow or other desired local responses). In some implementations, the transmitter 102 can be configured to transmit energy (e.g., the first energy $E_1$), in which a first portion of the energy is at a first frequency and/or charge configured to avoid causing a local response and a second portion of the energy is at a second frequency and/or charge configured to cause the desirable local response. In some implementations, the transmitter 102 can be configured to transmit the first portion of the energy and the second portion of the energy in any suitable combination, pattern, interval, sequence, and/or the like.

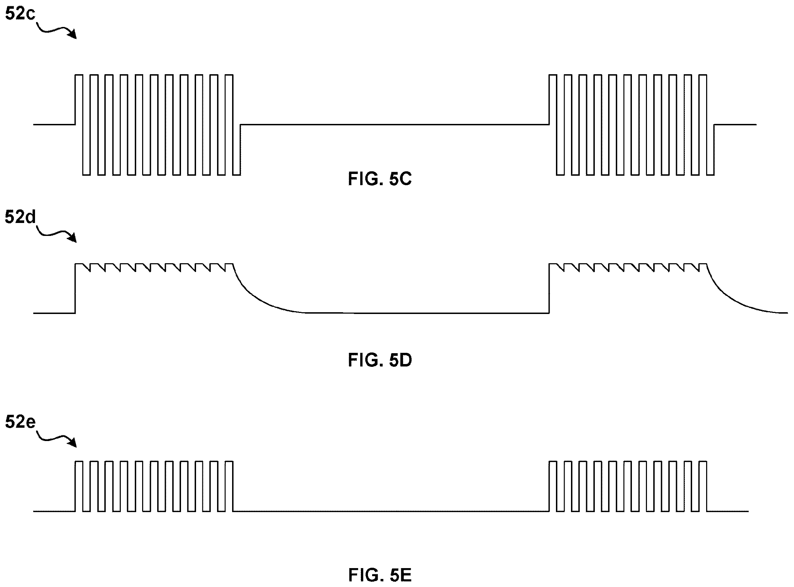

In some implementations, the predetermined waveform can include, for example, a sinusoidal waveform, a rectangular waveform, a triangular waveform, or any other suitable waveform (as described in further detail herein with reference to FIGS. 5C-5E). The predetermined waveform can otherwise include any suitable type of waveform. Operating parameters by which the transmitter 102 can be configured to transmit the energy can include, for example, pulse width, pulse frequency, current magnitude, current density, power magnitude, power density, and the like.

The transmitter 102 can be configured to transmit the energy by application of the output to a body of a subject at or with respect to a position, region, or location surrounding, encompassing, or adjacent to a position or location at which the power adapter 100 or the implant 104 are disposed (e.g., implanted) in the body, such as shown in FIG. 1A. For example, the transmitter 102 can be configured to transmit the energy by application of the output to the body, transcutaneously, such as along or with respect to a path (e.g., electrical path, conductive path) at least partially disposed internal to the body, and interconnecting the transmitter 102, the power adapter 100, and the implant 104. That is, the path can be defined, in part, by the body into which the transmitter 102 is configured to transmit the energy, such as by the portion of the body between the transmitter 102, power adapter 100, and implant 104.

The implant 104 represents an implant such as an implantable device, including, for example, an implantable electrical conductor, and/or the like ("implant" or "implantable device" or "implantable electrical conductor"). The implant 104 can be configured to be powered by and/or otherwise use energy received from an external device such as an external transmitter or power supply (e.g., transmitter 102), via a power adapter (e.g., power adapter 100), to perform a medical procedure in a body (e.g., in environment 101) of a subject, as described herein. In some implementations, the implant 104 can include an onboard energy source, energy storage device, and/or the like, such as a battery. Such a battery can, for example, store and/or be recharged by the energy received transcutaneously.

For example, in some instances, the implant 104 can be or include an implantable electrical conductor, such as of an implantable stimulation device, or stimulator, configured to operate in the body, and to be powered, via the power adapter 100, by an external device such as the transmitter 102. In these instances, the implantable stimulation device, or stimulator, can be or include, for example, a nerve stimulator, an artificial pacemaker, and/or the like. In other instances, the implant 104 can be or include an implantable electrical conductor, such as of a fluid conveyance device, or fluid conveyor, such as a pump or compressor (e.g., insulin pump), or a vacuum, suction, or depressurizing device. In other instances, the implant 104 can be or include an implantable electrical conductor, such as a sensor, transducer, monitor, and/or recorder, including, for example, an electrocardiography (ECG) sensor, a heart rate monitor, a Holter monitor, and/or the like. The implant can otherwise be or include any suitable type and number of implantable electrical conductors.

As shown in FIG. 1B, the implant 104 includes electrodes 19a and 19b, interconnected over conductor 18. The implant 104 can include an input and an output, such as at the electrode 19a and the electrode 19b, respectively. For example, the implant 104 can be configured to receive energy at the input (e.g., at the electrode 19a), and to provide energy at the output, (e.g., at the electrode 19b). Energy can be conveyed between the input (e.g., electrode 19a) and the output (e.g., electrode 19b) via an implantable electrical conductor (e.g., conductor 18) of the implant 104. The implant 104 can be configured to receive, transcutaneously and at the electrode 19a, energy from a transmitter such as transmitter 102. The energy can be received, for example, to power the implant 104, to control the implant 104 (e.g., as in performing a medical procedure), and/or the like.

In some implementations, the implant 104 can be configured to receive energy from the transmitter 102 via the power adapter 100. For example, in some instances, such as when the power adapter 100 is connected to the implant 104, as shown in FIG. 1A, the power adapter 100 can be configured to receive the first energy $E_1$ (e.g., having a first frequency, waveform and/or other characteristic) from the transmitter, for conversion of the first energy to the second energy $E_2$ (e.g., having a second frequency, waveform and/or other characteristic), and transfer of the second energy $E_2$, from the power adapter 100 and to the implant 104, such as by input to the implant 104 at the electrode 19a, such that the implant 104 receives the second energy $E_2$ (e.g., for output at electrode 19b). In some implementations, when the power adapter 100 is not connected to the implant 104 (e.g., as shown in FIG. 1B), the electrode 19a can receive the second energy $E_2$ directly. By connecting the power adapter 100 to the implant 104 and over the electrode 19a, the implant 104 can be retrofitted and/or adapted to receive the first energy $E_1$ rather than the second energy $E_2$. That is, when the power adapter 100 is connected to the implant 104, such as shown in FIG. 1A, the power adapter 100 can prevent the electrode 19a from directly receiving energy. The energy output by electrode 19b can be detected and/or received by the transmitter 102 (e.g., by a skin electrode (not shown in FIGS. 1A or 1B) to complete an electrical circuit including the transmitter 102, the housing 110 and the implant 104.

The electrodes 19a and 19b can each include one or more electrodes, electrical contacts, electrical terminals, and the like. The electrode 19a can include an input electrode and the electrode 19b can include an output electrode. For example, the electrode 19a can include an input electrode such as a receiving electrode, a pick-up electrode, and/or the like (referred to herein as "pick-up electrode"). In some implementations, such as those in which the implant 104 is a stimulation device, the electrode 19b can include an output electrode such as a stimulating or stimulation electrode, a stimulation lead, and/or the like (referred to herein as "stimulating electrode" or "stimulation electrode"). In some implementations, the electrode 19a can include and/or can be formed of a material such as a titanium (Ti), titanium-nitride (TiN), platinum-iridium (Pt-Ir) compound, and/or the like. In some implementations, the electrode 19b can include and/or can be formed of a material such as a platinum (Pt), iridium (Ir), a platinum-iridium (Pt-Ir) compound, or alloy, and/or the like. The conductor 18 can include any suitable electrical conductor, electrical lead, and/or conductive material over which the electrodes 19a and 19b can be interconnected. For example, the conductor 18 can include a path such as a conductive path or an electrical path configured to interconnect the electrodes 19a and 19b over the implant 104. The conductor 18 can include and/or can be formed of a material such as an inert or non-reactive material, or any other material suitable for use in a body of a subject, in accordance with embodiments described herein.

The housing 110 can be or can include any suitable type of housing or casing. For example, the housing 110 can include a housing such as an hermetically sealed casing, or can, configured to house or otherwise contain one or more circuits (e.g., circuit 120), and, having a feedthrough, inner contact (e.g., electric conductor), one or more mating features (e.g., grip mechanism assembly) configured to electrically and mechanically couple to and make contact with a pick-up electrode (e.g., electrode 19a of implant 104), and a sleeve (e.g., for mechanical and/or electrical protection). The housing 110 can be configured to at least partially house one or more circuits, including, for example, the circuit 120. The housing 110 can be configured to be coupled to an implant such as implant 104 for disposition, with implant 104 (and the circuit 120), in a body of a subject. The housing 110 can be configured to mechanically insulate the circuit 120 from the body, including, for example, from an environment in the body such as environment 101. For example, the housing 110 can be configured to insulate the circuit 120 from, for example, an environment such as environment 101 in the body of the subject, such as when the housing 110 is coupled to implant 104 and disposed in environment 101, such as by implantation with implant 104 in the body. The housing 110 can include any suitable housing capable of attaching, coupling, connecting, interconnecting, or otherwise being added, mechanically, electrically, and otherwise, to an implant such as implant 104, as described herein. The housing 110 can include any suitable type and number of components, such as including resistors, capacitors, transistors, diodes, inductors, an energy source, energy storage device, and/or the like. In some implementations, the housing 110 does not include an energy source, energy storage device, and/or the like, which can be or include, for example, a battery or other chemical source of energy. In other implementations, the housing can include an energy storage device (e.g., battery, energy storage capacitor, etc.) that can be used to power the implant 104 and/or can be recharged by receiving the transcutaneous transfer of energy, as described herein. In some implementations, the housing 110 can be or include, for example, a hermetically sealed can configured to at least partially house the circuit 120.

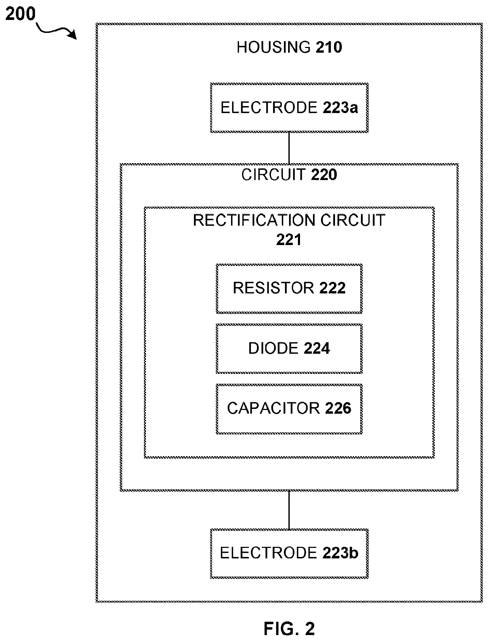

The circuit 120 can be or include a circuit such as an integrated circuit (IC), and/or the like. The circuit 120 can be configured to be electrically connected to an implantable device such as the implant 104 when the housing 110 is coupled to the implant 104, such as at the electrode 19a. For example, the circuit 120 can be configured to electrically connect to the implant 104, when the housing 110 is coupled to the implant 104, such as at a pick-up electrode (e.g., electrode 19a) of the implant 104, to enable the circuit 120 to provide energy (e.g., transformed power, conditioned signals) to the implant 104. The energy can be provided, by the circuit 120 and to the implant 104, via input to the implant 104 at the pick-up electrode (e.g., via a conductor or electric interface in electric communication with the electrode 19a). The circuit 120 can be configured to receive the energy (e.g., for conversion of the energy and transfer of the converted energy to implant 104) from a transmitter such as transmitter 102, as described herein. The circuit 120 can include various components, such as described herein with reference to FIG. 2.

As an example, in use, the power adapter 100 can be configured to be implanted, in a coupled or interconnected state with implant 104, in a body of a subject. For example, the power adapter 100 can be configured to be coupled to implant 104 by attachment of the housing 110 over a pick-up electrode (electrode 19a) of the implant 104. In some instances, the power adapter 100 can be configured to be retrofit to an existing implant in a body of a subject, such as the implant 104. For example, the power adapter 100 can be configured to be mated to the existing implant such as by crimping, or the like. Once the power adapter 100 is implanted in the body with the implant 104, operating parameters, including, for example, stimulation parameters, and the like, can be set (e.g., at transmitter 102), as described herein. Accordingly, the power adapter 100 - along with the transmitter 102 and the implant 104 - can be configured for use, such as by the subject of the body (in which the power adapter 100 is implanted with the implant 104).

In other implementations, the power adapter 100 can be integral to the implant 104. For example, in some implementations, the power adapter 100 can be provided as part of or embedded in the implant 104, such as in a pre-coupled or -interconnected state with the implant (e.g., via interconnection to electrode 19a). Similarly stated, in such implementations, the functions of the power adapter 100 (as described herein) can be part of and/or integrated into the implant. In such implementations, a separate power adapter 100 is not needed and/or used to receive the transcutaneous energy transfer.

In some implementations, such as those in which the implant 104 is a stimulation device and the electrode 19b includes an output electrode such as a stimulating electrode, the power adapter 100 can be configured to convert the first energy $E_1$ (e.g., from transmitter 102) to the second energy $E_2$, for input of the second energy $E_2$ to the implant 104 to enable the implant 104 in performing a medical procedure. In such implementations, the medical procedure can include, for example, a medical procedure in which the implant 104 is configured to provide stimulation, activation, excitation, and the like ("stimulation") of tissue, nerves, or muscles in a body of a subject. In such implementations, the implant 104 can be configured to perform the medical procedure in the body via output of the second energy $E_2$ at the electrode 19b. In such implementations, the second energy $E_2$ can include, for example, a sequence of low frequency pulses or bursts and/or a sequence of high frequency pulses or bursts. Specifically, the second energy $E_2$ can include, for example, interlaced delivery of low and high frequency energy, stimulation, bursts, and/or pulses. The medical procedure can be performed, for example, to activate a cutaneous receptor, a muscle, and/or a nerve of the body.

FIG. 2 is a schematic block diagram depicting a power adapter 200, in accordance with an embodiment. As shown, the power adapter 200 includes a housing 210 and a circuit 220 at least partially disposed in the housing 210. The power adapter 200 can be configured to be coupled or interconnected to an implant (e.g., the implant 104) for disposition in a body, such as to operate in an environment (e.g., the environment 101) of and internal to the body. The circuit 220, when the housing 210 is coupled to an implant (e.g., the implant 104) and implanted in a body, can be configured to electrically interconnect (e.g., via an electrode 223b) to a stimulating electrode of the implant. The power adapter 200 can be structurally and/or functionally similar to other power adapters (e.g., the power adapter 100) shown and described herein.

The circuit 220 includes a rectification circuit 221 and an electrode 223a (e.g., pick-up electrode). The rectification circuit 221 can be or include, for example, a halfwave-rectification circuit or a fullwave-rectification circuit. For example, in some instances, the rectification circuit 221 can include a resistor 222, a diode 224, and a capacitor 226. While not shown or described with respect to FIG. 2, in other implementations (e.g., as shown and described with respect to 9A, 9B and/or 10), the circuit can include another capacitor and/or an inductor to provide protection at frequencies used with respect to Magnetic Resonance (MR) devices such as, for example, Magnetic Resonance Imaging (MRI) devices. The rectification circuit 221 can be configured to selectively convert received energy (e.g., received from the transmitter 102 via the electrode 223a). For example, the rectification circuit 221 can be configured to convert first energy by rectification of the first energy to provide second energy (e.g., via the electrode 223b). In some instances, the second energy can be substantially positive DC or substantially negative DC. As an example, the rectification circuit 221 can be configured to convert and filter received signals in a manner similar to that of an amplitude modulation (AM) receiver.

The capacitor 226 can be or include, for example, a direct current (DC) blocking capacitor. The capacitor 226 can be configured to maintain a level of charge balance of the rectification circuit 221. For example, the capacitor 226 can be configured to provide charge balancing of energy transmitted from the rectification circuit 221. In some implementations, such as those in which the implant 104 is a stimulation device, a type or characteristic of the capacitor 226 can be chosen, for example, based on a characteristic (e.g., operating condition) such as tissue-electrode capacitance, such as of a pick-up electrode (e.g., electrode 19a) and a stimulating electrode (e.g., electrode 19b) of the implant 104, with respect to tissue internal to a body of a subject (e.g., in environment 101). In such implementations, the capacitor 226 can effectively be connected in series with the pick-up electrode and the stimulating electrode. In a serial connection of capacitors, the capacitor with the least amount of capacitance (i.e., the capacitor with the smallest measure of capacitance) determines the combined capacitance of the capacitors (e.g., which is substantially equal to the capacitance of the capacitor with the least relative amount of capacitance). Accordingly, the capacitor 226 can be chosen or configured to have a particular value or measure of capacitance to not decrease the overall capacitance of the path (e.g., interconnecting the capacitor 226, the pick-up electrode, and the stimulating electrode) based on the effective capacitance of the tissue-electrode capacitance of the pick-up electrode and the stimulating electrode.

As an example, where the tissue-electrode capacitance is approximately 4 microfarad ($\mu$F), the capacitor 226 can be chosen or configured to have a value or measure of capacitance of approximately 4 $\mu$F, or greater. In this example, the value of the capacitor 226 can be chosen or configured based on the tissue-electrode capacitance of the tissue internal to the body and the pick-up electrode (e.g., electrode 19a) and the stimulating electrode (e.g., electrode 19b) of the implant 104. In some implementations, the capacitor 226 can be chosen or configured to have a value or measure of capacitance that does not decrease but supports and/or maintains an overall capacitance of the conductive path (e.g., the path interconnecting a pick-up electrode with a stimulating electrode) of the implant 104.

The diode 224 can be or include, for example, a rectifying diode. In some implementations, the diode 224 can be or include a rectifying diode such as a Schottky diode, a silicone diode, and/or the like. In some implementations, a type or characteristic of the diode 224 can be chosen, for example, based on a characteristic such as a magnitude of a voltage drop (e.g., in a forward direction) over the diode 224. For example, the type of the diode 224 can be chosen to reduce a magnitude of the voltage drop over the diode 224. In this example, the type of the diode 224 can be chosen to be or include a Schottky diode (e.g., instead of a silicon diode) to reduce the magnitude of the voltage drop over the diode 224 (e.g., compared to that of the silicon diode), and to thereby achieve a higher pick-up ratio (e.g., compared to that of a silicone diode). In some implementations, a type of the diode 224 can be chosen based on or to facilitate any suitable characteristic, such as amount of leak current, amount of back leak current, a discharge rate (e.g., of capacitor 226) between applied electrical bursts, and/or the like. For purposes of the present disclosure "pick-up" ratio refers to the amount of energy received by the implant relative to the amount of energy sent by the external transmitter. For example, a pick-up ratio of 0.5 indicates that the amount of energy received by the implant is approximately half the amount of energy sent by the external pulse transmitter.

The resistor 222 provides a discharge path (from rectification circuit 221) for the capacitor 226. In some implementations, a type or characteristic of the resistor 222 can be chosen, for example, based on a characteristic of the rectification circuit 221 including, for example, a discharge path characteristic of the rectification circuit 221. For example, the resistor 222 can be chosen to have a measure or value of resistance greater than an effective resistance of the diode 224, to prevent bypass (e.g., by electrical current) of the diode 224 in use (e.g., of the power adapter 200 with an implant such as implant 104). In some implementations, a type or characteristic of the resistor 222 can be chosen, for example, based on an applied frequency or frequency range of the energy (e.g., electrical signals, electrical bursts) from transmitter 102, a burst repetition frequency of the applied frequency or frequency range of the energy, a burst duration of the applied frequency or frequency range of the energy, and/or the like.

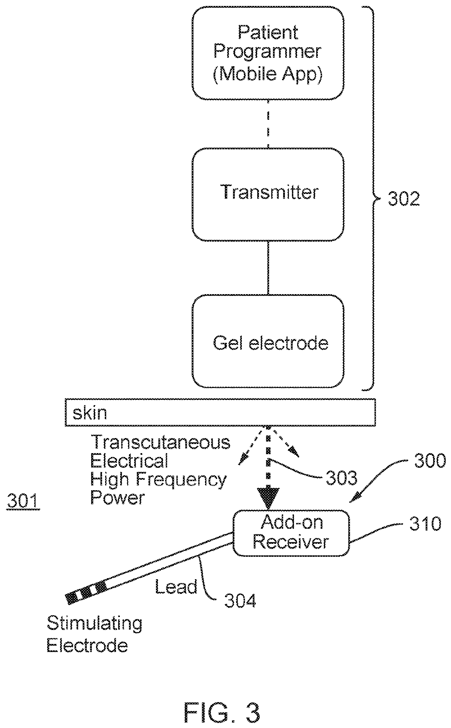

FIG. 3 is a schematic block diagram depicting an example use of a power adapter 300 in conjunction with a transmitter 302, in accordance with an embodiment. As shown, the power adapter 300 includes a housing 310 (labeled "add-on receiver") and a circuit (not shown) at least partially disposed in the housing 310. The power adapter 300 can be structurally and/or functionally similar to other power adapters (e.g., 100, 200) described herein.

The transmitter 302 can be configured to send or otherwise provide energy to power adapter 300 (for powering and/or supplying energy to implant 304) via path 303. In some implementations, the electrical pulse generator (e.g., transmitter 102, transmitter 302) can include, for example, a power supply. The path 303, along which the energy is received, transferred, and applied, can include, for example, a portion of the body of the subject between the transmitter 302 (e.g., at a gel and/or cloth electrode of the transmitter (not shown)) and the power adapter 300 (when disposed with implant 104 in the body).

For example, the power adapter 300, the housing 310, and the circuit can be structurally and/or functionally similar to the power adapter 100, the housing 110, and the circuit 120, respectively, as described herein. The power adapter 300 can be configured to be coupled, via the housing 310, to an implant such as implant 304 for disposition in a body with implant 304, such as beneath skin and in environment 301 of the body. The power adapter 300 can be configured to be attached or coupled to implant 304 such that the pick-up electrode of implant 304 is electrically insulated from the environment 301 (e.g., when implant 304 and power adapter 300 are implanted in a body). The power adapter 300 can be configured to receive energy from the transmitter 302 for conversion and transfer to implant 304, and application, via a stimulating electrode of implant 304, to a target site or object in the body.

The transmitter 302 can be structurally and/or functionally similar to the transmitter 102, as described herein. For example, the transmitter 302 can include an external transmitter (labeled "transmitter") and a patch (not shown) including one or more gel electrodes (labeled "gel electrode"). In some implementations, the external transmitter can include, for example, a high frequency transmitter. While shown in FIG. 3 as gel electrodes, in some implementations, the patch can include, for example, a gel patch, a hydrogel patch, a cloth patch, and/or the like, including, for example, electrodes such as gel electrodes, hydrogel electrodes, cloth electrodes, and/or the like. In some implementations, the patch can include a disposable patch. The transmitter 302 can be configured to transmit energy transcutaneously into the body of a subject (e.g., for receipt by the circuit disposed in the housing 310), such as by application, via the patch, of the output of the transmitter 302 to the body.

Implant 304 can be structurally and/or functionally similar to implant 104, as described herein. For example, implant 304 can include an electrical conductor or lead (labeled "lead"), a stimulating electrode (labeled "stimulating electrode"), and a pick-up electrode (not shown), over which the power adapter 300 can be attached or coupled, such as described herein with reference to FIG. 1, and shown in FIG. 3. The lead of implant 304 can include, for example, a conductive path interconnecting the stimulating electrode and the pick-up electrode. The lead of implant 304 can be or include, for example, an electrical conductor such as a coiled wire (Pt-Ir) conductor disposed within a silicone sheath, or tubing. For example, the lead of implant 304 can be insulated (e.g., from tissue in the environment 301) by the silicone tubing and by silicone backfill disposed in and configured to close the tubing at each end. Implant 304 can be configured to receive, transcutaneously and via the power adapter 300 (e.g., disposed at the pick-up electrode of implant 304), energy (e.g., electrical signal, electromagnetic signal, magnetic signal) from the transmitter of the transmitter 302. For example, implant 304 can be configured to receive the energy to apply, via the stimulating electrode, a stimulus (e.g., electrical bursts, electrical pulses) to a target site or object in a body of a subject. In some implementations, implant 304 can include, for example, three or more stimulating electrodes (e.g., such as the electrode 19b).

In use, the power adapter 300 can be configured to receive, transcutaneously from the transmitter 302, transdermal high frequency bursts of energy (e.g., electrical energy). The energy can be received at, or can otherwise include, for example, a first frequency of between about 10 kHz and 100 kHz, or greater. In other instances, the first frequency can be between 100 kHz and 3 megahertz (MHz). In yet other instances, the first frequency can be 10 MHz or less and/or any other suitable frequency. The received energy can be converted, by the power adapter 300, to a form suitable for use in providing stimulation, activation, or excitation (e.g., of tissue, nerve, muscle) in a body of a subject. For example, the received energy can be converted, by the power adapter 300, to a second energy (e.g., stimulation current) having a second frequency less than the first frequency, such as, for example about 1 kHz. In other implementations, the second frequency can be between 1 kHz and 10 kHz. In yet other implementations, the second frequency can be between 500 Hz and 30 kHz. The energy conversion can include, for example, rectification and charge balancing via the power adapter 300. The converted energy can be transferred, from the power adapter 300 to a stimulating electrode of the implant 304, for application to a target in the body (e.g., nerve) at the stimulating electrode.

As an example, the implant 304 can be or include a lead such as a flexible electrical conductor having a length of approximately 15 cm and a diameter of approximately 1.2 mm. The stimulating electrode of the implant 304 can be positioned at or near a target object in the body, such as a nerve, or the like. The pick-up electrode of the implant 304 can be covered by attachment of the power adapter 300 to the end of the implant 304 at which the pick-up electrode is disposed. The target object can include any suitable point, region, or part of interest, such as a nerve (e.g., peroneal nerve, peripheral nerve, etc.). In some implementations, the implant 304 can include, for example, one or more stimulating electrodes having dimensions in the range of approximately 1 mm in length. In some implementations, where the implant 304 includes three or more stimulating electrodes, the stimulating electrodes can be spaced along the lead of the implant 304 at a spacing of approximately 1 mm apart. In some implementations, one or more of the stimulating electrodes of the implant 304 can be manufactured or assembled by coiling of an electrical conductor (e.g., the lead of the implant 304) on the outside of the silicone tubing (e.g., silicone sheath) and at the end of the lead, such as shown in FIG. 3. A conductive surface of the stimulating electrode (e.g., at the stimulation end of the implant 304) can be configured to be in contact with surrounding tissue in the environment 301 when implanted (e.g., with the power adapter 300) in the body. In some implementations, the implant 304 can include, for example, an anchor (e.g., hook, tines) having a diameter of approximately 1.5 mm. The anchor can be configured to fix the implant 304 in position, or otherwise prevent lead migration in the environment 301 upon implantation and positioning of the implant 304 with the power adapter 300 in a body of a subject. For example, the anchor can include a silicone anchor having four prongs or hooks and can be disposed at the stimulation end of the implant 304.

In some implementations, the transmitter 302 can optionally be configured to be used or programmed for use via software (e.g., residing on a device external to the transmitter). For example, the software can reside or otherwise be hosted on any suitable type of compute device (e.g., mobile device, tablet computer, server). For example, the software can be executed at a compute device to generate and send signals (e.g., including commands) to the transmitter 302 for execution (e.g., at the transmitter 302), and the transmitter 302 can be configured to receive, from the compute device, one or more of the signals, including, for example, a signal corresponding to a command configured to be executed at the transmitter 302. The signals can include, for example, machine- or processor-readable code and/or instructions configured to be stored on and/or executed at the transmitter 302. In some implementations, the code can include instructions configured to be executed at the transmitter 302, such as to set or specify one or more operating parameters, stimulation parameters, and/or the like, of and/or at the transmitter 302. For example, one or more of the operating parameters of the transmitter 302 can include a particular stimulation routine to be applied (e.g., via the implant 304), a particular stimulation intensity to be applied (e.g., transcutaneously to the body), an applied frequency or frequency range of the energy to be applied, and so on. The software can be configured for use, for example, by a user or operator such as a clinician, a patient, and/or the like.

In some implementations, the software by which the transmitter 302 can optionally be configured to be used or programmed for use can be stored, for example, at a compute device such as a tablet compute device. In some instances, the compute device can be configured to communicate with the transmitter 302 via a communications link such as a Bluetooth Low Energy (BLE) communications link, or the like. In some instances, the software can be configured to enable access to data including, for example, patient demographic information, session data, patient stimulation profiles, and the like. In some instances, the software can reside and/or otherwise can be hosted for use via a smartphone platform (e.g., iOS, Android). In some instances, the software can include, for example, a mobile app. In some implementations, the software can be configured to enable, for example, use tracking, system error or fault notification, and/or the like. In some implementations, the software can be configured to control various functions of the transmitter 302, including, for example, selection of a stimulation program or routine (e.g., as pre-defined by a user such as a clinician), stimulation activation and deactivation (e.g., turning the transmitter 302 on and off), increase or decrease (applied) stimulation intensity, and so on. In some implementations, the software can be configured to provide (e.g., via a display, transducer such as a speaker) an indication (e.g., visual, auditory) as to operating status, such as with respect to selected stimulation program, selected stimulation intensity level, good or bad electrode connection, among other types of indications of errors or operating status.

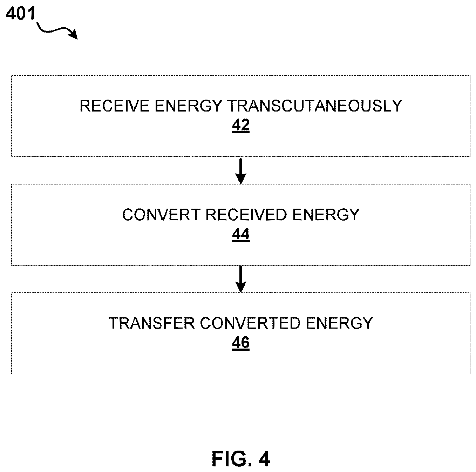

FIG. 4 is a flowchart depicting a method 401 of using a power adapter, in accordance with an embodiment. The power adapter can be structurally and/or functionally similar to any of the power adapters (e.g., 100, 200, and/or 300) described herein.

At 42, the method 401 includes receiving (e.g., via the power adapter 100, 200, and/or 300), transcutaneously and from an electrical pulse generator (e.g., the transmitter 102 and/or 302), first energy at a first frequency and/or first waveform. At 44, the method 401 includes converting, via a rectification circuit (e.g., the rectification circuit 221), the first energy to a second energy. In some implementations, the second energy can have a second frequency different from the first frequency and/or a second waveform different from the first waveform. At 46, the method 401 includes transferring, from the rectification circuit, the second energy to a stimulating electrode (e.g., the electrode 19b shown in FIGS. 1A and 1B) of an implantable electrical conductor (e.g., the implant 104 and/or 304) such that the implantable electrical conductor applies, at the second frequency and via the stimulating electrode, the second energy to a target internal to a body (e.g., of a subject). The target internal to the body can include, for example, a nerve, a region in the body, and/or the like.

In some implementations, the second energy can be transferred from the rectification circuit (e.g., the rectification circuit 221) to a pick-up electrode (e.g., the electrode 19a) of the implantable electrical conductor (e.g., the implant 104), for subsequent transfer and routing via the implantable electrical conductor (e.g., the conductor 18 of the implant 104) to the stimulating electrode (e.g., the electrode 19b), and application, at the stimulating electrode, to a target nerve internal to the body. In some implementations, the second energy can be transferred from the rectification circuit to the implantable electrical conductor, and in particular, the stimulating electrode, to enable application of the second energy to the target internal to the body. In some implementations, the first energy can include, for example, alternating current. In some implementations, the second energy can include, for example, pulsating direct current. In some implementations, the first frequency can include, for example, a frequency in the range of about 30 kHz and 100 kHz. When the apparatus is not coupled to the implantable electrical conductor (e.g., via the housing 110, 210, and/or 310), the pick-up electrode of the implantable electrical conductor can be configured to receive, transcutaneously (e.g., from the electrical pulse generator), third energy at substantially the second frequency and/or second waveform.

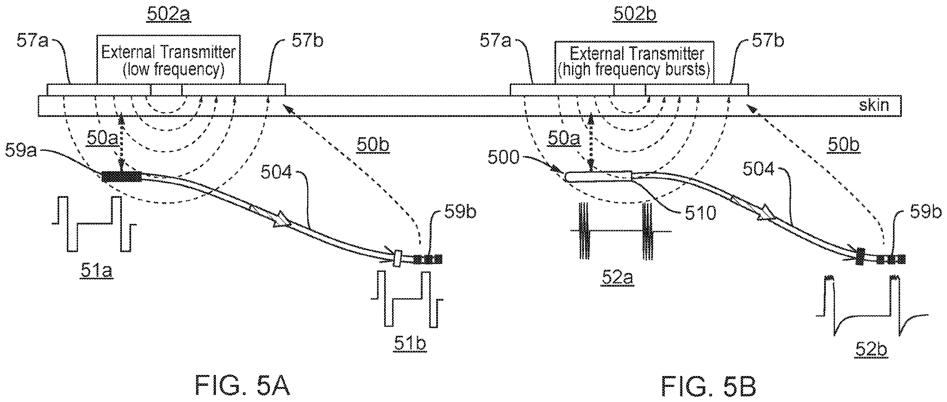

FIGS. 5A and 5B are schematic diagrams depicting an effect of using a power adapter 500 in conjunction with a transmitter (e.g., the transmitter 502b) and the implant 504, in accordance with an embodiment. The power adapter 500 can be structurally and/or functionally similar to other power adapters (e.g., the power adapter 100, 200, and/or 300) described herein. The implant 504 can be structurally and/or functionally similar to the implants or implantable electrical conductors (e.g., the implant 104 and/or 304) described herein.

With reference to FIG. 5A, transmitter 502a (labeled "External Transmitter (low frequency)") can be configured to apply transcutaneous stimulation (e.g., first energy) via an electrode patch 57a (e.g., disposed at a skin surface) into a body of a subject. The transmitter 502a can be or include, for example, a low frequency external transmitter, and/or the like, configured to operate in conjunction with the implant 504 (e.g., without the power adapter 500). The transmitter 502a can be structurally and/or functionally similar to any of the transmitters (e.g., the transmitter 102), as described herein.

The transmitter 502a can be configured to transmit the energy by application (e.g., via the electrode patch 57a) of the output to the body (e.g., at a skin surface of the body), transcutaneously, such as along or with respect to a path (e.g.,

electrical path, conductive path) at least partially disposed internal to the body, and interconnecting the transmitter 502a and the implant 504. The path can include, for example, the electrode patch 57a, a first portion of the body 50a, the implant 504 (e.g., via the electrodes 59a and 59b), a second portion of the body 50b, an electrode patch 57b, and the transmitter 502a. A portion of the applied transcutaneous stimulation (e.g., 10%-20%) can be picked up or received by the implant 504, at electrode 59a, and can be transferred and/or routed, to electrode 59b and along the implant 104 (e.g., via the conductor 18). The electrode 59a can include, for example, a pick-up electrode. The electrode 59b can include, for example, a stimulating electrode.

In some implementations, the implant 504 can include insulation such as a silicone backfill and tubing, disposed about a lead body (e.g., the conductor 18 shown in FIGS. 1A and 1B) of the implant 504, such that energy (e.g., electrical pulses received via the electrode 59a) can be transmitted efficiently to the conductive surfaces of the stimulation electrode contacts (e.g., of the electrode 59b), where the electrical current can then be applied to a target such as a target peripheral nerve, or any other suitable site in the body, as described herein. In some implementations, the lead body (e.g., the conductor 18 shown in FIGS. 1A and 1B) of the implant 504 can include, for example, a Pt-Ir lead.

In some implementations, the energy frequency 51a at the pick-up electrode and the energy frequency 51b at the stimulating electrode can be similar, or substantially equal or identical. In some implementations, the waveform can also be similar, or substantially equal or identical, with the exception of the signal amplitude. The transmitter 502a can be configured to apply and deliver energy transcutaneously at a low applied frequency or frequency range (e.g., below 10 kHz) for stimulation at the low applied frequency at and by the electrode 59b.

With reference to FIG. 5B, transmitter 502b (labeled "External Transmitter (high frequency bursts)") can be configured to send or transmit first energy (e.g., energy including high frequency bursts) via electrode patch 57a (e.g., disposed at a skin surface) into a body of a subject, such as described herein. The transmitter 502b can be or include, for example, a high frequency external transmitter, and/or the like, configured to operate in conjunction with the implant 504 via power adapter 500. The transmitter 502b can be structurally and/or functionally similar to transmitters (e.g., the transmitter 102 and/or 302) described herein.

The transmitter 502b can be configured to send the first energy at a frequency of approximately 10 kHz - 100 kHz, to avoid causing sensation in the body of the subject. The transmitter 502b can be configured to send the first energy at a frequency to avoid causing direct activation of the nerves about the location of application of the transcutaneous stimulation to the body. The transmitter 502b can be configured to transmit the energy by application (e.g., via the electrode patch 57a) to the body (e.g., at a skin surface of the body), transcutaneously, such as along or with respect to a path (e.g., electrical path, conductive path) at least partially disposed internal to the body, and interconnecting the transmitter 502b, the power adapter 500, and the implant 504. The path can include, for example, the electrode patch 57a, a first portion of the body 50a, the power adapter 500 (e.g., via the electrode 123 and/or 223a in FIGS 1 and 2, respectively), the implant 504 (e.g., via the electrodes 59a and 59b), a second portion of the body 50b, an electrode patch 57b, and the transmitter 502b.

A portion of the applied transcutaneous stimulation such as between approximately 10%-20% (e.g., from the transmitter 502b) can be picked up by the pick-up electrode of the power adapter 500, in the form of the first energy 52a (e.g., having a first frequency and/or having a first waveform) and converted, by a rectification circuit (e.g., the rectification circuit 221) of a circuit (e.g., the circuit 120 and/or 220) of the power adapter 500 (e.g., at least partially disposed in the housing 510 of the power adapter 500), to second energy 52b (e.g., having a second frequency and/or having a second waveform). The second energy 52b can include, for example, low frequency bursts, high frequency bursts, and/or the like. The second energy 52b can be routed to the electrode 59b for application, via one or more electrodes at or of the electrode 59b, to a target such as a target peripheral nerve, or any other suitable site in the body, such as to treat pain. In some implementations, the second energy 52b can include, for example, a sinusoidal waveform, a rectangular waveform, a triangular waveform, or the like. For example, the power adapter 500 (via the circuit disposed in the housing 510) can be configured to operate in a manner similar to that of an AM radio receiver, by demodulating energy including signals such as high frequency bursts (e.g., carrier wave) and detecting the low frequency (e.g., modulated) signal. As such, the power adapter 500 can be configured to be retrofit and/or adapted for use in or with an implant (e.g., the implant 504) normally configured to receive energy at a first frequency (e.g., a low frequency) and/or having a first waveform, such that the implant can receive energy at a second frequency (e.g., low frequency pulses, high frequency bursts) and/or having a second waveform.

In some implementations, the rectification circuit of the circuit at least partially disposed in housing 510 of the power adapter 500 can include a rectifying diode (e.g., the diode 224) oriented in a cathodic orientation, such as shown in FIG. 5B, such that cathodic stimulation is provided via the stimulating electrode (of the implant 504). In some implementations, the rectification circuit of the circuit at least partially disposed in housing 510 of the power adapter 500 can include a rectifying diode (e.g., the diode 224) oriented in a cathodic orientation such that cathodic stimulation is provided via the stimulating electrode (of the implant 504). The nerve (e.g., sensory, motor) activation threshold in the cathodic orientation (e.g., negative pulse delivered to the stimulating electrode) is lower than that of an anodic orientation of the rectifying diode (e.g., the diode 224) as it causes more effective depolarization of the cell membrane and subsequent activation of the nerve. In some implementations, the housing 510 can be or include a hermetically sealed housing made of Titanium. The first energy (e.g., current at first frequency) applied by the transmitter 502b can be returned, transcutaneously and from the stimulating electrode, to the transmitter in the form of the second energy (e.g., current at second frequency) to complete the electrical circuit. For example, the rectifying diode (e.g., the diode 224) can be oriented to be connected to the stimulating electrode of the implant 504. In other implementations, the rectifying diode (e.g., the diode 224) can be oriented in an anodic orientation such that anodic stimulation is provided via the stimulating electrode (of the implant 504).

In some implementations, the transmitter 502b can provide the first energy having a first waveform that can include, for example, multiple sets of bursts or pulses, which in turn can be rectified by the power adapter 500 into the second energy having a second waveform. FIGS. 5C-5E are example waveforms 52c, 52d, and 52e, respectively, illustrating potential waveforms used with respect to a power adapter, in accordance with different implementations. As shown in FIG. 5C, the transmitter 502b can provide energy having the waveform 52c, which can be provided similar to the first energy 52a. A characteristic of the waveform 52c can include, for example, a first frequency and/or waveform, such as a rectangular waveform, or the like. More specifically, the waveform 52c is shown with two sets of bursts or pulses having square or rectangular waveforms with the first frequency and that alternate in phase (e.g., a positive phase and a negative phase). The waveform 52c can be used to provide the first energy 52a to the power adapter 500. The power adapter 500 can then convert the first energy 52a to second energy 52b having a second frequency and/or second waveform.

FIGS. 5D and 5E show waveforms 52d and 52e, respectively, that are examples of waveforms of second energy 52b (e.g., as input by the power adapter 500 to the electrode 59a, and applied by the implant 504 via output at the electrode 59b). Specifically, the waveform 52d of FIG. 5D is a rectified version of the waveform 52c of FIG. 5C (e.g., using envelope detection rectification). The waveform 52d is shown with two sets of bursts or pulses having a non-symmetric waveform and a single phase (e.g., one of a positive phase or a negative phase). More specifically, the square wave bursts of the waveform 52c are rectified to produce the square waveform 52d, which effectively is a square waveform having a lower frequency than the square wave bursts of the waveform 52c. As another example, the waveform 52e of FIG. 5E can be produced using simple rectification of the waveform 52c. The predetermined waveform 52e is shown with two sets of bursts or pulses having a rectangular waveform and a single phase (e.g., one of a positive phase or a negative phase). For example, the waveform 52e can include the positive components of the waveform 52c with the negative portions of the waveform 52c removed. In some instances, the frequency of the waveform 52e (e.g., of the second energy) can be similar or substantially equal or identical to the frequency of the waveform 52c (e.g., of the first energy). While the waveforms 52c, 52d, and 52e are each shown with two sets of bursts or pulses, it should be understood that the waveforms 52c, 52d, and 52e can include any number of bursts or pulses. Moreover, while the bursts or pulses are particularly shown in FIGS. 5C-5E, it should be understood that they are provided by way of example only and not limitation.

FIG. 5F is a graph illustrating the relationship between charge per burst and frequency when applied transcutaneously to an individual, according to an embodiment. As shown in FIG. 5F, the predetermined frequency or range of frequencies (e.g., at which the first energy is output from the transmitter 502b) can include, for example, a frequency or range of frequencies in the range of approximately 10 kHz to 100 kHz. The predetermined frequency or range of frequencies can otherwise include a frequency or range of frequencies and the range of energy and/or charge at which the energy output from the transmitter 102 can be applied, such as to a body of a subject, without causing a response, or stimulation ("response"), such as a local motor response or sensation, in and by the body. For example, the predetermined frequency or range of frequencies and the amount of energy and/or charge can be chosen or determined to achieve a targeted response (labeled "Targeted response") as a function of frequency with respect to a magnitude of the applied energy. The magnitude of the applied energy can be specified, for example, such as in terms of a magnitude of electrical current, or in terms of a delivered electrical charge, which is measured in Coulombs.

As illustrated in FIG. 5F, as the frequency increases, the amount of energy and/or charge that can be applied to the individual without an undesirable local response can also increase. Line A illustrated in FIG. 5F is an example frequency at which the transmitter 502a of FIG. 5A can transmit the first energy 51a to an implant that does not include a power adapter such as the power adapter 500. Line B illustrated in FIG. 5F is an example frequency at which the transmitter 502b of FIG. 5B can transmit the first energy 52a to an implant (e.g., the implant 504) that is coupled to or includes the power adapter 500. In some instances, when at higher frequencies, there can be a larger margin (e.g., the "Operational Window") between the energy sufficient to result in a response of the targeted tissue near the implant and the energy sufficient to result in an undesirable local response under the skin electrodes.

While the transmitters 502a is described above as transmitting the first energy 51a having a relatively low frequency and the transmitter 502b is described above as transmitting the first energy 52a having a relatively high frequency, in some embodiments, a transmitter can be configured to transmit energy that includes any suitable combination of the energy 51a (e.g., the relatively low frequency) and the energy 52a (e.g., the relatively high frequency). In such implementations, the transmitter can transmit the energy in any suitable pattern, combination, sequence, interlaced or non-interlaced series, time-dependent bursts or pulses, random bursts or pulses, and/or the like. In some instances, the relatively low frequency energy can be configured to result in and/or otherwise cause a desirable local response such as, for example, increased blood flow or other desirable response within a region of the body adjacent and/or near the transmitter, while the relatively high frequency energy can be received by the power adapter and transmitted to the implant, as described above.

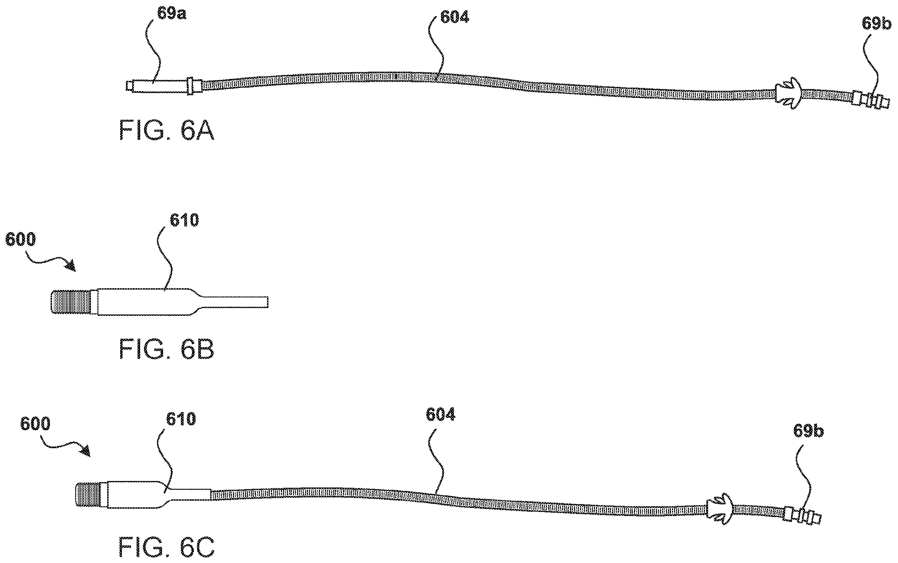

FIGS. 6A-6F depict various views of a power adapter 600 and/or an implant 604, in accordance with an embodiment. The power adapter 600 can be structurally and/or functionally similar to other power adapters (e.g., 100, 200, 300, and/or 500) shown and described herein. The implant 604 can be structurally and/or functionally similar to other implants (e.g., 104, 304, and/or 504) shown and described herein. For example, the implant 604 can include a pick-up electrode 69a and a stimulating electrode 69b, such as shown in FIG. 6A.

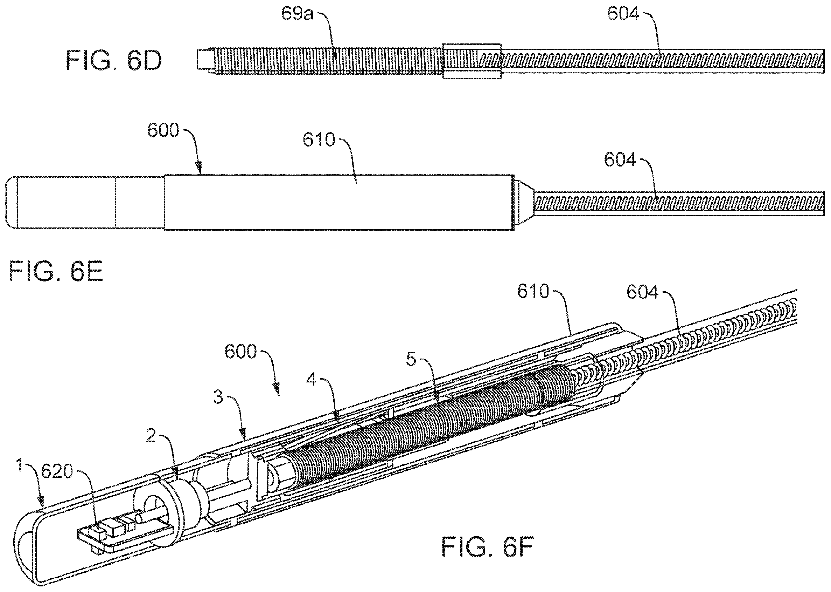

In some implementations, the housing 610 can be configured to be coupled, for example, to, on, and/or over implant 604, such that the housing 610 at least partially covers an end of implant 604, such as shown in FIGS. 6E and 6F. For example, the housing 610 can be configured to be coupled on and over implant 604 to at least partially cover (e.g., non-hermetically) one or more of the electrodes, such as a pick-up electrode, of the implant 604, as described herein. In this example, in covering one or more of the electrodes of the implant 604, the housing 610 can be configured to insulate (e.g., electrically insulate) the one or more (e.g., covered) electrodes from surrounding tissue (e.g., as in environment 101) when disposed in a body with implant 604. In some implementations, the one or more covered (e.g., by housing 610) electrodes of implant 604 can include, for example, a pick-up electrode. In some implementations, the housing 610 can be configured to be coupled to, on, and over implant 604 with a retainment force of approximately 6.5 Newtons (N).

The pick-up electrode 69a of the implant 604 is shown in FIG. 6D. As shown in FIG. 6E, the power adapter 600 can be attached on and over the pick-up electrode of the implant 604. As shown in FIG. 6F, circuit 620 can be at least partially disposed in the housing 610, where the housing 610 includes, for example, a housing (1), configured to function as a pick-up electrode of the power adapter 600. The housing 610 can be configured to hermetically seal the circuit 620 inside the housing (1). Further, as shown in FIG. 6F, the power adapter 600 can include a feed-through conductor (2) through which (converted) energy from the circuit 620 can be transferred to the stimulating electrode of the implant 604. Further, as shown in FIG. 6F, the power adapter 600 can include electrical conductors (4). The electrical conductors (4) can be, for example, press-fit against the pick-up electrode of the (5) of the implant 604. The housing 610 can be configured to couple to the implant 604 and can fit over the pick-up electrode of the implant 604, upon coupling of the housing 610 to the implant 604. The housing 610 can include a silicone sleeve (3), to electrically insulate the pick-up electrode from surrounding tissue (e.g., when the housing 610 is coupled to the implant 604 and disposed in a body). The silicone sleeve (3) can be configured to provide a friction or retainment force to the coupling between the housing 610 and the implant 604 upon coupling of the housing 610 to the implant 604. For example, the silicone sleeve (3) can be configured to apply pressure and friction to the coupling or interface between the power adapter 600 and the implant 604 upon coupling of the housing 610 to the implant 604.

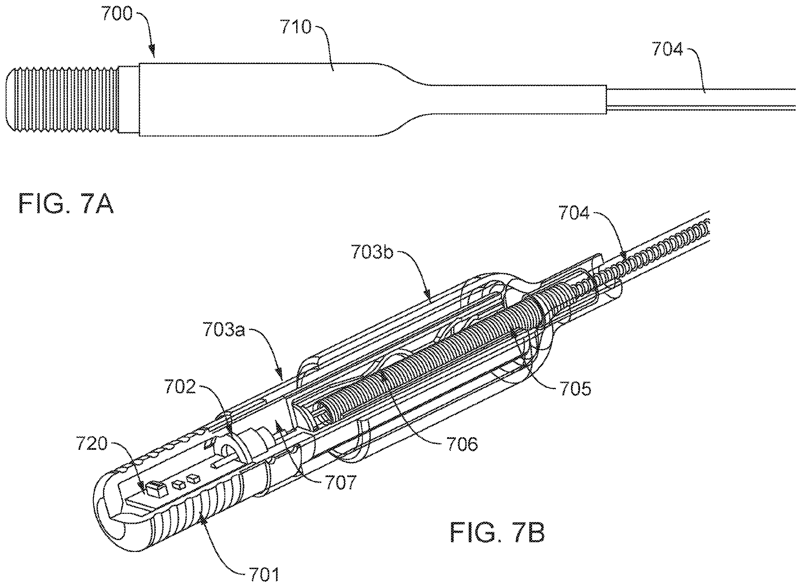

FIGS. 7A and 7B depict a side view and a partial cross-sectional view, respectively, of a power adapter 700 and a portion of an implant 704, in accordance with an embodiment. The power adapter 700 can be structurally and/or functionally similar to other power adapters (e.g., 100, 200, 300, 500, and/or 600) shown and described herein. The implant 704 can be structurally and/or functionally similar to other implants (e.g., 104, 304, 504, and/or 604) shown and described herein. For example, the implant 704 can include a pick-up electrode and a stimulating electrode (not shown), as described above with reference to the implant 604 of FIG. 6A.

In some implementations, a housing 710 of the power adapter 700 can be configured to be coupled, for example, to, on, and/or over implant 704, such that the housing 710 at least partially covers an end of implant 704, such as shown in FIGS. 7A and 7B. For example, the housing 710 can be configured to be coupled on and over implant 704 to at least partially cover (e.g., non-hermetically) one or more of the electrodes, such as a pick-up electrode, of the implant 704, as described herein. In this example, in covering one or more of the electrodes of the implant 704, the housing 710 can be configured to insulate (e.g., electrically insulate) the one or more (e.g., covered) electrodes from surrounding tissue (e.g., as in environment 101) when disposed in a body with implant 704. In some implementations, the one or more covered (e.g., by housing 710) electrodes of implant 704 can include, for example, a pick-up electrode. In some implementations, the housing 710 can be configured to be coupled to, on, and over implant 704 with a retainment force of approximately 6.5 Newtons (N).

As shown in FIG. 7B, the power adapter 700 can be attached on and over a pick-up electrode 705 of the implant 704. A circuit 720 can be at least partially disposed in the housing 710 and, in conjunction with the pick-up electrode 705, can be configured to function as a pick-up electrode of the power adapter 700. The housing 710 can be configured to hermetically seal the circuit 720 inside the housing 710. As shown, the housing 710 can include a first sleeve 703A and a second sleeve 703B. The first sleeve 703A can be, for example, a sleeve, cover, housing, etc. formed from any suitable material. For example, the first sleeve 703A can be formed from materials such as thermoplastic polyurethane (e.g., Tecothane), polyether ether ketone (PEEK), and/or the like. Similarly, the second sleeve 703B can be a sleeve, cover, housing, etc. formed from any suitable material (e.g., a material similar to or different from the material of the first sleeve 703A). For example, the second sleeve 703B can be formed from a material such as silicone and/or the like. In some embodiments, at least one of the first sleeve 703A and/or the second sleeve 703B can be configured to electrically insulate the pick-up electrode 705 from surrounding tissue (e.g., when the housing 710 is coupled to the implant 704 and disposed in a body). Further, the first sleeve 703A and the second sleeve 703B - alone or in combination - can be configured to provide a friction or retainment force to the coupling between the housing 710 and the implant 704. For example, the sleeve(s) 703A and/or 703B can be configured to apply pressure and friction to the coupling or interface between the power adapter 700 and the implant 704 upon coupling of the housing 710 to the implant 704.

As shown in FIG. 7B, the power adapter 700 can include a feed-through conductor 702 through which (converted) energy from the circuit 720 can be transferred to the stimulating electrode of the implant 704. The power adapter 700 can further include electrical conductors 706. The electrical conductors 706 can be, for example, press-fit against the pick-up electrode 705 of the implant 704. The electrical conductors 706 can be electrically connected to the feed-through conductor 702, thereby allowing the electrical conductors 706 to transmit electric power between the feed-through conductor 702 and the pick-up electrode 705 of the implant 704. A space 707 within the housing 710 at or around an interface between the feed-through conductor 702 and the electrical conductors 706 can be filed with epoxy and/or silicone and configured to electrically insulate the interface therebetween. Accordingly, the power adapter 700 can be structurally and/or functionally similar to the power adapter 600.

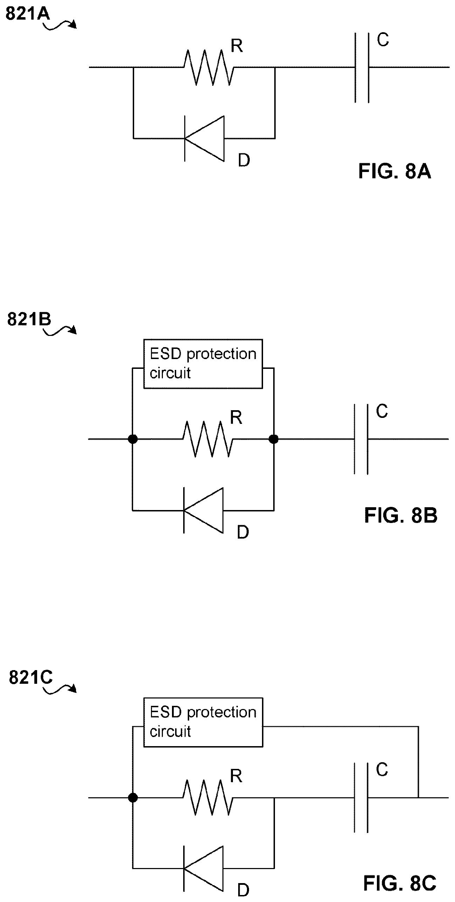

FIG. 8A is a schematic diagram depicting a circuit 821A of a power adapter, in accordance with an embodiment. The circuit 821A can be structurally and/or functionally similar to other circuits or a portion of other circuits (e.g., the circuit 221) described herein.

As shown, the circuit 821A includes a capacitor C (e.g., the capacitor 226) in series with a resistor R (e.g., the resistor 222), which is in parallel with a diode D (e.g., the diode 224). The diode D can include a rectifying diode. The capacitor C can include a DC blocking capacitor, as described above with respect to capacitor 226 in FIG. 2. In some embodiments, the capacitor C can be disposed on either side of the diode D. The diode D can be oriented in cathodic orientation or in anodic orientation. For example, in the cathodic orientation, when the circuit 821A is connected to an implant (e.g., the implant 104), a cathode of the diode D can be connected to the implant (e.g., at the electrode 19a). As another example, in the anodic orientation, when the circuit 821A is connected to an implant (e.g., the implant 104), an anode of the diode D can be connected to the implant (e.g., at the electrode 19a of the implant 104). The resistor R can be disposed in parallel to the diode to enable discharge of the capacitor C during positive phase of the pulse (e.g., second energy).

FIGS. 8B and 8C are schematic diagrams depicting individual circuits 821B and 821C, respectively, of a power adapter, in accordance with an embodiment. The circuits 821B and 821C can be configured to provide electrostatic discharge protection (ESD) via an ESD protection circuit. The circuits 821B and 821C can otherwise be structurally and/or functionally similar to other circuits or a portion of other circuits (e.g., the circuit 221) described herein.

As shown, the circuits 821B and 821C can include a capacitor C (e.g., the capacitor 226) in series with a resistor R (e.g., the resistor 222) and a diode D (e.g., the diode 224) - the resistor R is in parallel with the diode D. Moreover, each circuit 821B and 821C can include an electrostatic discharge (ESD) protection circuit, such as shown in FIG. 8B. For example, as shown in FIG. 8B, the circuit 821B can include the ESD protection circuit connected in parallel with the diode D (and the resistor R). Accordingly, the ESD protection circuit in the circuit 821B can be configured to provide protection over the diode D. As another example, as shown in FIG. 8C, the circuit 821C can include the ESD protection circuit connected in parallel with the diode D and the capacitor C (and the resistor R). In some implementations, the ESD protection circuit can include, for example, a diode such as a Zener diode, a transient volt suppressor (TVS) diode, bidirectional Zener diodes (e.g., two diodes connected in series front to front or back to back) and/or the like. The ESD protection circuit can be configured to reduce an exposure to risk of accidental electrostatic discharge such as during manufacturing and implantation, and further, reduces the need for other ESD protection.

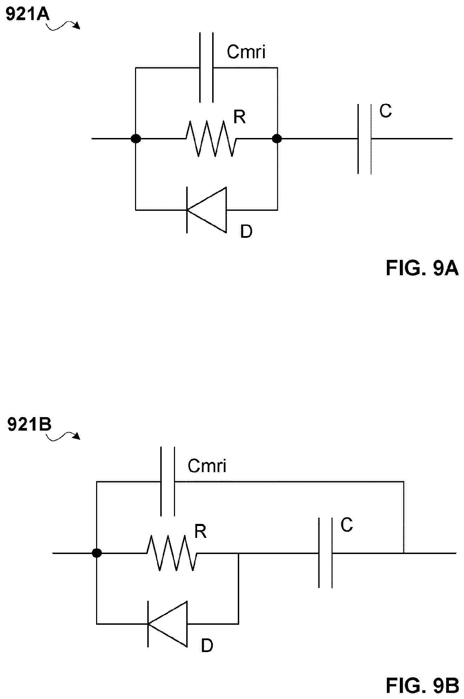

FIGS. 9A and 9B are schematic diagrams depicting individual circuits 921A and 921B, respectively, of a power adapter, in accordance with an embodiment. The circuits 921A and 921B can be structurally and/or functionally similar to other circuits or a portion of other circuits (e.g., the circuit 221) described herein.

As shown, each circuit 921A and 921B includes a capacitor C (e.g., the capacitor 226) in series with a resistor R (e.g., the resistor 222) and a diode D (e.g., the diode 224) - the resistor R is in parallel with the diode D. Moreover, each circuit 921 can include a capacitor Cmri configured to provide magnetic resonance imaging (MRI) protection. For example, as shown in FIG. 9A, the circuit 921A can include the capacitor Cmri connected in parallel with the diode D (and the resistor R). As another example, as shown in FIG. 9B, the circuit 921B can include the capacitor Cmri connected in parallel with the diode D and the capacitor C (and the resistor R). Accordingly, the capacitor Cmri, connected as such in either of the circuits 921A and 921B can be configured to provide, at low frequencies (50 kHz), relatively high impedance. Moreover, at higher frequencies (e.g., 64 MHz, 128 MHz) such as in MRI machines, the capacitor Cmri can be configured to provide low impedance and effectively will prevent rectification by effectively shorting (i.e., short-circuiting) the diode D. Thus, only non-rectified current will be delivered to the stimulating electrode (e.g., from either of the circuits 921A and 921B). Moreover, non-rectified current at 64 MHz or 128 MHz will not activate the nerve (unlike the rectified current) and will not cause any unintended stimulation and/or unpleasant sensation during the MRI procedure. For example, the capacitor Cmri be chosen to have a capacitance of approximately 100 picoFarads (pF), and, as such, can have an impedance, at 50 kHz of approximately 30,000 ohms; at 64 MHz = 25 Ohm; and at 128 MHz = 12 Ohm. The aforementioned frequencies are MRI frequencies (for 1.5 T and 3.0 T MRI machines respectively), which will bypass the rectifying circuit via the Cmri short circuit (e.g., 921A and 921B). Accordingly, at these frequencies, the circuits 921A and 921B are configured to not provide rectified pulses to the stimulating electrode of the implant (e.g., the implant 104).

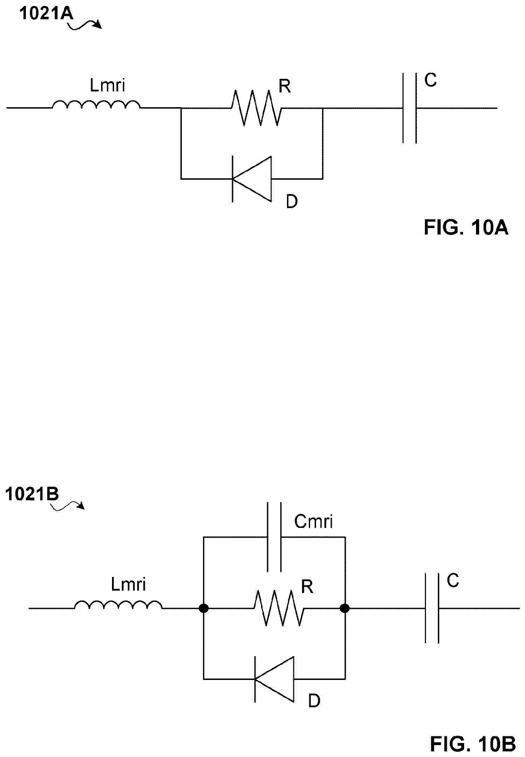

FIGS. 10A and 10B are schematic diagrams depicting individual circuits 1021A and 1020B, respectively, of a power adapter, in accordance with an embodiment. The circuits 1021A and 1021B can be structurally and/or functionally similar to other circuits or a portion of other circuits (e.g., the circuit 221) described herein.

As shown, each circuit 1021A and 1021B includes a capacitor C (e.g., the capacitor 226) in series with a resistor R (e.g., the resistor 222) and a diode D (e.g., diode 224) - the resistor R is in parallel with the diode D. As shown in FIG. 10A, the circuit 1021A can include an inductor Lmri disposed and connected in series with the rest of the circuit. Compared to adding a capacitor (e.g., Cmri) to the circuit (e.g., as shown in FIGS. 9A and 9B), the inductor Lmri can be configured to block higher frequencies, reduce current via the receiver, and can prevent undesired stimulation and also heating (e.g., of the power adapter 100 and/or the implant 104) due to the current flow. For example, the inductor Lmri be chosen to have an inductance of approximately 5 nanohenries (nHy) to provide 2 Ohms at 50 kHz; 2 kOhm at 64 MHz; and 4 kOhm at 128 MHz. In some implementations, the inductor Lmri can include dimensions of approximately 2.5 mm x 2.5 mm x 3.8 mm. The aforementioned frequencies are MRI frequencies that will be blocked by the inductor, which is capable of blocking the MRI frequencies in the circuit 1021A (e.g., as described above with reference to the circuits 921A and 921B). Accordingly, at these frequencies, the circuit 1021A is configured to not provide pulses to the stimulating electrode of the implant (e.g., the implant 104), thereby providing protection to the patient when in an MRI machine.

While the circuit 1021A is shown in FIG. 10A as including the inductor Lmri as an alternative to the capacitor Cmri included in the circuits 921A and 921B, in some embodiments, a circuit can include both an inductor and a capacitor (e.g., a LC circuit). For example, as shown in FIG. 10B, the circuit 1021B includes a capacitor Cmri and an inductor Lmri, each of which can be configured to provide magnetic resonance imaging (MRI) protection alone or in combination. As described above with reference to the circuit 1021A, the inductor Lmri in the circuit 1021B is connected in series with the rest of the circuit. Thus, at least one of the capacitor 1021A and/or the inductor 1021B can limit, prevent, and/or substantially prevent the circuit 1021B from providing pulses to the stimulating electrode of the implant (e.g., the implant 104), thereby providing protection to the patient when in an MRI machine.

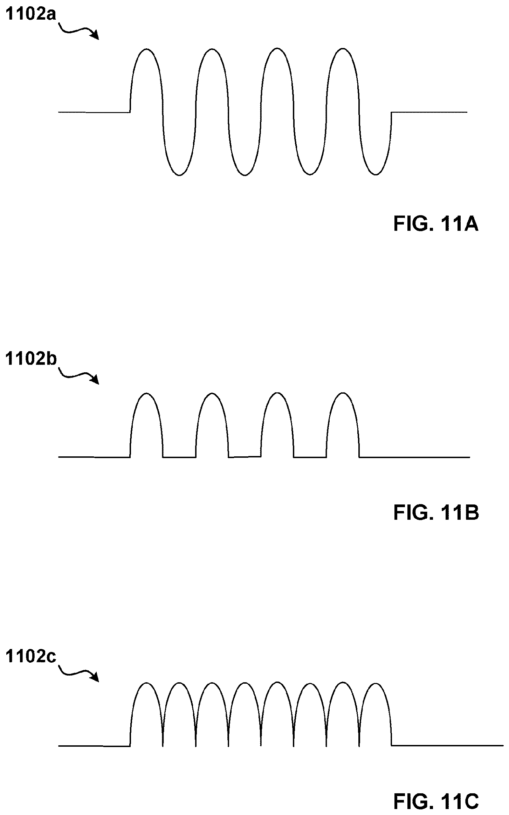

FIGS. 11A-11C are waveforms illustrating potential waveforms used with respect to a power adapter, in accordance with an embodiment. Any of the power adapters described herein can be used with, can receive, can convert, and/or can output energy having any suitable characteristic or set of characteristics, which can include, for example, one or more characteristics associated with waveform. For example, FIG. 11A illustrates a waveform 1102a, in accordance with an embodiment. The waveform 1102a can be, for example, a non-rectified waveform associated with and/or otherwise having alternating current. As described in detail above, a transmitter such as those described herein can be configured to generate and provide energy (e.g., a first energy) to a power adapter. In some instances, the first energy can have a waveform similar to or substantially the same as the waveform 1102a shown, for example, in FIG. 11A.

The power adapters described in detail herein can be configured to receive a first energy and to convert and output a second energy. For example, the power adapters can include one or more circuits having any suitable components, as described in detail above with reference to specific embodiments. In some implementations, a power adapter can be configured to convert energy received from the transmitter (e.g., the first energy) to an energy (e.g., a second energy) having one or more different characteristics. For example, in some embodiments, the power adapter and/or at least a portion thereof can be configured to rectify the first energy received from the transmitter such that a second energy having a rectified waveform (e.g., a halfwave rectified) waveform or a fullwave rectified waveform) is transferred to, for example, a pick-up electrode of an implant. In some instances, the rectification can be, for example, a one-way rectification (also referred to as halfwave-rectification). For example, FIG. 11B illustrates a waveform 1102b resulting from, for example, a one-way or halfwave rectification of the waveform 1102a. In other instances, the rectification can be, for example, a two-way rectification (also referred to as fullwave-rectification). For example, FIG. 11C illustrates a waveform 1102c resulting from, for example, a two-way or fullwave rectification of the waveform 1102a.

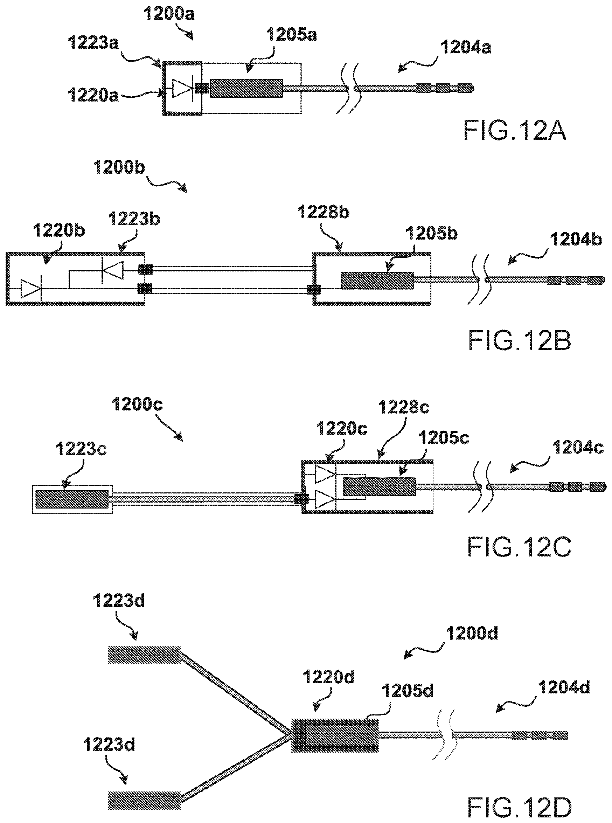

FIGS. 12A-12D are schematic diagrams depicting power adapters, in accordance with various embodiments. As described above with reference to FIGS. 11A-11C, in some implementations the power adapters described herein can be configured to rectify an energy transcutaneously received from a transmitter. More specifically, FIG. 12A illustrates a power adapter 1200a coupled to an implant 1204a. The power adapter 1200a and the implant 1204a can be similar in at least form and/or function to any of the power adapters and implants, respectively, described in detail herein. The power adapter 1200a can include a circuit 1220a and one or more electrodes 1223a that is/are configured to receive energy from the transmitter (e.g., as described above with reference to the electrode 123 and/or 223a). In the embodiment shown in FIG. 12A, the power adapter 1200a and/or the circuit 1220a can be configured to perform, for example, one-way or halfwave rectification on the energy (e.g., a first energy) received from the transmitter and can provide energy having the one-way of halfwave rectified waveform (e.g., shown in FIG. 11B) to a pick-up electrode 1205a of the implant 1204a (e.g., a second energy).

FIG. 12B illustrates a power adapter 1200b coupled to an implant 1204b, in accordance with an embodiment. The power adapter 1200b and the implant 1204b can be similar in at least form and/or function to any of the power adapters and implants, respectively, described in detail herein. As shown, the power adapter 1200b can include a circuit 1220b, one or more proximal electrodes 1223b, and a distal electrode 1228b. The electrodes 1223b and 1228b can be configured to receive energy from the transmitter, as described in detail above. In the embodiment shown in FIG. 12B, the power adapter 1200b can be configured as a lead or the like having the circuit 1220b disposed at or near the proximal end and the distal electrode 1228b disposed at or near the distal end. Moreover, the power adapter 1200b and/or the circuit 1220b can be configured to perform, for example, two-way or full-wave rectification on the energy (e.g., a first energy) received from the transmitter and can provide the two-way or fullwave rectified energy (e.g., a second energy) to a pick-up electrode of the implant 1204b. For example, in the example shown in FIG. 12B, the proximal electrode 1223b and the distal electrode 1228b can be in electrical communication with the transmitter and configured to transfer energy therebetween (e.g., via two electrical connections, wires, interconnects, etc.). In some embodiments, the circuit 1220b can include, for example, two or more diodes that can enable the power adapter 1200b and/or the circuit 1220b to perform the two-way or fullwave rectification on the energy received from the transmitter (e.g., a first energy). As such, the power adapter 1200b can be configured to provide two-way or fullwave rectified energy to a pick-up electrode 1205b of the implant 1204b (e.g., a second energy).

While the power adapter 1200b is shown and described as including the circuit 1220b at or near the proximal end and the distal electrode 1228b at or near the distal end, in other embodiments, a power adapter configured to perform two-way of fullwave rectification on energy received from a transmitter can have any suitable arrangement. For example, FIG. 12C illustrates a power adapter 1200c coupled to an implant 1204c, in accordance with an embodiment. In this example, the power adapter 1200c includes a circuit 1220c and a distal electrode 1228c at or near the distal end of the power adapter 1200c and a proximal electrode 1223c at or near the proximal end of the power adapter 1200c. In some implementations, the power adapter 1200c can be similar in at least function to the power adapter 1200b and, as such, can be configured to provide two-way of fullwave rectified energy to a pick-up electrode of the implant 1204c. In some embodiments, providing the circuit 1220c at or near the distal end of the power adapter 1200c can allow for a single electrical connection between the proximal electrode 1223c and the distal electrode 1228c (e.g., rather than two electrical connections, as shown in FIG. 12B).

FIG. 12D illustrates a power adapter 1200d coupled to an implant 1204d, in accordance with an embodiment. In this example, the power adapter 1200d includes a circuit 1220d and a distal electrode (not shown in FIG. 12D) at or near the distal end of the power adapter 1200d, as described above with reference to the power adapter 1200c shown in FIG. 12C. In the example shown in FIG. 12D, the power adapter 1200d can include a pair of proximal electrodes 1223d at or near the proximal end of the power adapter 1200d. In some implementations, the power adapter 1200d can be similar in at least function to the power adapter 1200b and/or 1200c and, as such, can be configured to provide two-way rectified energy to a pick-up electrode of the implant 1204d. In some implementations, including various arrangements of one or more proximal electrodes (e.g., the proximal electrodes 1223d) can allow the power adapter 1200d to be used with transmitters having various shapes and/or sizes.

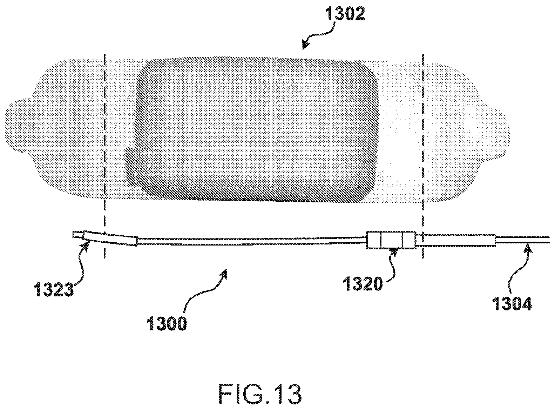

FIG. 13 illustrates a power adapter 1300 coupled to an implant 1304, and a transmitter 1302 configured to provide energy transcutaneously to the power adapter 1300, in accordance with an embodiment. As described above with reference to, for example, the power adapters 1200b, 1200c, and/or 1200d, the power adapter 1300 shown in FIG. 13 can be configured to perform two-way rectification on the energy received from a transmitter 1302. More particularly, the power adapter 1300 can be configured as a lead or the like that can be coupled to the implant 1304 as described in detail above. For example, the power adapter 1300 can be configured as a lead having a proximal electrode 1323 disposed at or near a proximal end of the lead and a circuit 1320 at or near a distal end of the lead.

In some embodiments, the lead can have a length of about 7.0 centimeters (cm). In other embodiments, the lead can be longer than 7.0 cm or can be shorter than 7.0 cm. In some embodiments, the length of the lead and/or power adapter 1300 can be at least partially based on a size and/or shape of the transmitter 1302 used therewith. For example, as shown in FIG. 13, the arrangement of the power adapter 1300 can be such that the proximal electrode 1323 is at least partially aligned with a first patch, a first side, and/or other suitable portion (e.g., a first portion) of the transmitter 1302 and the circuit 1320 and/or an electrode of the circuit 1320 (not shown in FIG. 13) is at least partially aligned with a second patch, second side, and/or other suitable portion (e.g., a second portion ) of the transmitter 1302. As such, the power adapter 1300, the transmitter 1302, and a portion of the body disposed therebetween can form a circuit and/or at least a portion of a circuit, thereby allowing the power adapter 1300 to perform two-way rectification on the energy (e.g., a first energy) received from the transmitter 1302. Moreover, with the power adapter 1300 coupled to, for example, a pick-up electrode of the implant 1304, the power adapter 1300 can be configured to provide two-way rectified energy (e.g., a second energy) to the implant 1304, as described in detail herein.

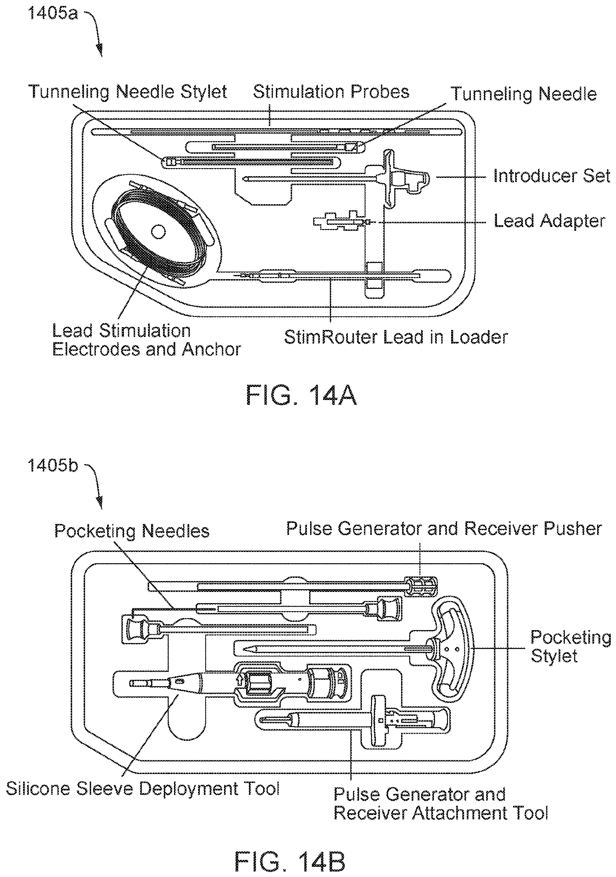

FIG. 14A is a schematic diagram depicting a kit 1405A including an implant, in accordance with an embodiment. As shown, the kit 1405A can include a lead adapter (labeled "Lead Adapter"), an implant (labeled "StimRouter Lead in Loader"), a tunneling needle stylet, stimulation probes, a tunneling needle, an introducer set, and one or more lead stimulation electrodes, and an anchor. The kit 1405A can also include a power adapter (not shown in FIG. 14A) that can be structurally and/or functionally similar to other power adapters (e.g., 100, 200, 300, 500, and/or 600) shown and described herein. The implant can be structurally and/or functionally similar to other implants (e.g., 104, 304, 504, and/or 604) shown and described herein. While the kit 1405A is shown as including seven or more discrete devices, other arrangements and/or configurations can include any number of devices and/or implements, in accordance with embodiments of the present disclosure

The kit 1405A represents a tool set including various implements and tools by which to facilitate disposition of the implant in a body of a subject.

The lead adapter can include a lead adapter configured to couple the implant (e.g., 104, 304, 504, and/or 604) to a transmitter (e.g., transmitter 102) such as during an intraoperative implantation procedure. When provided as part of the kit 1405A, the implant can include electrodes or probes, and be provided with an energy (e.g., signal, power) input end (e.g., at pick-up electrode) and an energy (e.g., signal, power) output end (e.g., at stimulating electrode, transducing end, sensing end), such as described herein. The implant can be provided in a loading or deployment device, or loader, configured to facilitate implantation of the implant in a body.

The loading or deployment device can be configured to maintain the implant in a sterile condition before and during end-use, and to reduce a risk of contamination during implantation of the implant (with the power adapter) in a body. The loading or deployment device can be configured to facilitate implantation of the implant (e.g., with the stimulating electrode end being the leading end).

The introducer set can include, for example, an incision-forming tool, a hollow tube (e.g., through which to dispose the power adapter and the implant in a body of a subject), and a seal. For example, the introducer set can include a trocar including an obturator, a tube such as a cannula, and a medical seal. The tunneling needle and the tunneling needle stylet can include a tunneling needle configured to facilitate access to a body, such for subsequent implantation of the implant (e.g., and the power adapter) in the body.

The anchor can include, for example, a silicon anchor. The anchor can otherwise include an anchor formed of any suitable material, such as a non-reactive or inert material, and the like. The anchor can be configured to fix the implant (e.g., along with the power adapter) in position in a body when disposed in the body. For example, the anchor can include a 4-pronged anchor configured to prevent or reduce lead migration after implantation. The kit 1405A can otherwise include any other suitable tool or implement for facilitating access to a body of a subject, and disposition (e.g., via implantation) of the power adapter and the implant in the body, in accordance with embodiments disclosed herein. For example, the kit 1405A can include tools and implements (provided and supplied in various conditions) such as listed in Table 1, below.

**Table 1: Tools and Implements**

| Components | | # included | Sterile |
| --- | --- | --- | --- |
| Implantable Lead (StimRouter Lead in Loader | | 1 | Yes |
| Stimulation Probes | | 2 | Yes |
| Stimulation Cables (yellow) | | 2 | Yes |
| Introducer Set 9 Fr | | 1 | Yes |
| Lead Adapter | | 1 | Yes |
| Tunneling Needle | | 1 | Yes |
| Tunneling Needle Stylet | | 1 | Yes |
| Pack of 4 Gel Electrodes | | 1 | No |
| Gel Electrode Cable (black) | | 1 | No |
| Procedure Manual | | 1 | No |

FIG. 14B is a schematic diagram depicting a kit 1405B including a power adapter, in accordance with an embodiment. The kit 1405B represents a tool set including various implements and/or tools that may be used to facilitate the coupling and/or attachment of a power adapter to an implant. As shown, the kit 1405B can include "Pocketing Needles," a "Pocketing Stylet," a "Silicone Sleeve Deployment Tool," a "Pulse Generator and Receiver Pusher," and a "Pulse Generator and Receiver Attachment Tool." The kit 1405B can also include a power adapter (not shown in FIG. 14B) that can be structurally and/or functionally similar to any of the power adapters described herein (e.g., the power adapters 100, 200, 300, 500, and/or 600) and/or an implant (not shown in FIG. 14B) that can be structurally and/or functionally similar to any of the implants described herein (e.g., the implants 104, 304, 504, and/or 604). While the kit 1405B is shown as including a number of discrete devices, other arrangements and/or configurations can include any number of devices and/or implements, in accordance with embodiments of the present disclosure

In some implementations, the pocketing needles and the pocketing stylet can be configured to facilitate access into a body. More specifically, the pocketing needles and the pocketing stylet can be used to access an implant (e.g., that is not already coupled to a power adapter) that is previously inserted into the body. The pulse generator and receiver pusher can be used to advance a power adapter to the implant disposed in the body and the pulse generator and receiver attachment tool can be used to attach the power adapter to the implant (e.g., physically and electrically attach). The silicone sleeve deployment tool can be used to apply a silicone sleeve over at least a portion of the power adapter and/or the implant (e.g., as described above with reference to the sleeves 703A and 703B shown in FIG. 7B).

In some instances, the kit 1405A and the kit 1405B can be sold, packaged, and/or shipped together as a combined unit or kit. In other instances, the kit 1405A can be sold, packaged, and/or shipped separately from the kit 1405B.

Any of the implantable devices, power adapters, and/or the like can be configured to receive electrical energy from any suitable external transmitter. In some implementations, an external transmitter (also referred to as an "external pulse transmitter" or "EPT") can be configured to provide bursts, pulses, and/or flows of electrical charge in a body of a subject, which can be received or "picked-up" by any one or a combination of the power adapters and/or implants described herein. In other implementations, an EPT can be, for example, a transcutaneous stimulator that can be configured to provide stimulation (e.g., bursts, pulses, and/or flows of electrical charge) to a target area or nerve of a subject without the use of an implantable device. For example, such an EPT can be a transcutaneous electrical nerve stimulator (TENS) device, a neuromuscular electrical stimulator (NMES) device, and/or the like.

In some embodiments, a suitable EPT can be substantially similar to the transmitter 102 described above with reference to FIGS. 1A and 1B. In some instances, it can be desirable for an EPT to transcutaneously provide energy with a predetermined set of characteristics. In some implementations, the predetermined set of characteristics can include, for example, a frequency or range of frequencies, a voltage or range of voltages, a current or range of currents, a waveform or range of waveforms, a symmetric or asymmetric pulse, etc. Moreover, in some implementations, the predetermined set of characteristics can be selected to reduce or avoid adverse sensory or motor stimulation (e.g., an undesirable local response), undesirable heating of the EPT or energy loss in the form of heat, undesirable charge buildup in the skin and/or tissue (e.g., a load circuit), undesirable charge imbalances, and/or the like.

Examples of suitable transmitters (or portions, features, and/or aspects thereof) and the use and/or implementation of such transmitters is provided below. While specific embodiments and/or implementations are provided below, it should be understood that they have been presented by way of example only and not limitation. While embodiments and/or implementations are described as being used with an implantable device (such as any of those described above), it should be understood that a transmitter can be substantially similar in form and/or function to those described but configured to provide transcutaneous stimulation to a target area of a subject without the additional use of an implantable device. Similarly, it should be understood that a transmitter can be used in conjunction with an implant with or without a power adapter (such as those described above). While embodiments and/or implementations are described as having specific features, other embodiments having additional features or other combinations of features are possible and other implementations are contemplated within the scope of this disclosure.

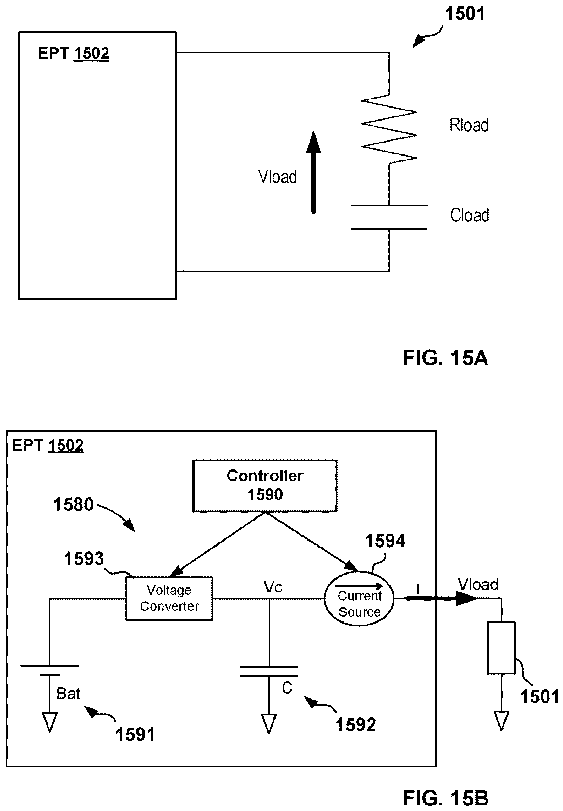

For example, FIGS. 15A-15B are various schematic diagrams illustrating at least portions of an external pulse transmitter (EPT) 1502 electrically connected to a load 1501, according to an embodiment. The EPT 1502 can be any suitable transmitter such as any of those described herein. For example, in some embodiments, the EPT 1502 and/or portions or aspects thereof can be similar to and/or substantially the same as the transmitter 102 and/or portions or aspects thereof. Accordingly, such similar portions of the EPT 1502 may not be described in further detail herein. In some implementations, the EPT 1502 can be configured to transcutaneously provide electrical energy to an implanted device such as any of the power adapters and/or implants described herein. Moreover, in the embodiment shown in FIG. 15A, the EPT 1502 can be configured to reduce, minimize, and/or otherwise at least partially control an amount of heat associated with and/or generated by the use of the EPT 1502, as described in further detail herein.

As shown in FIG. 15A the EPT 1502 can include one or more circuits that is configured to provide stimulation, energy, voltage, current, etc., to the connected load 1501. In some implementations, for example, the EPT 1502 is in electrical contact with the skin of a subject wearing the EPT 1502. A portion of the skin and tissue of the patient, therefore, can form the load 1501 electrically connected to the EPT 1502. More specifically, the skin and tissue of the subject have an impedance that can be represented as a capacitor ($C_{load}$) in series with a resistor ($R_{load}$) and the EPT 1502 can be connected to the load 1501 such that a stimulation, energy, voltage, current, etc. generated by the EPT 1502 results in a flow of current across the load 1501 having a load voltage ($V_{load}$), as shown in FIG. 15A. In some instances, for example, the load 1501 (e.g., the skin and tissue of the patient) can have an impedance represented as a 300 Ohm resistor in series with a 30 nanoFarad (nF) capacitor.

For example, FIG. 15B illustrates at least a portion of a stimulating circuit 1580 included in the EPT 1502 that is configured to provide stimulation, energy, current, etc. to the load 1501. The stimulating circuit 1580 includes at least a battery 1591, a capacitor 1592, a voltage converter 1593, and a current source 1594. The EPT 1502 and/or the stimulating circuit 1580 also includes a controller 1590 configured to control at least the voltage converter 1593 and the current source 1594. The controller 1590 can be any suitable hardware-based, software-based, and/or firmware-based controller configured to control the operation of at least the voltage converter 1593 and the current source 1594. In some embodiments, for example, the controller 1590 can include a microprocessor and/or the like configured to execute a set of instructions and/or code stored, for example, in a memory (not shown in FIG. 15B).

The battery 1591 can be any suitable battery configured to output a known and/or desired voltage. Said a different way, the battery 1591 can be any suitable size or type of battery and can be configured to be and/or to act as a voltage source in the stimulating circuit 1580. The voltage converter 1593 is electrically connected to the battery 1591 and is configured to up-convert the voltage provided from the battery 1591 to a desired higher voltage (e.g., a compliance voltage (Vc)) suitable for driving a desired amount of current through the load 1501. In addition, the voltage converter 1593 can supply a flow of high voltage current to charge the capacitor 1592 of the EPT 1502. In some implementations, the compliance voltage Vc can be determined based at least in part on an amount of current produced by the current source and an impedance of an attached load 1501. For example, in some implementations, the compliance voltage Vc can be between about 20 volts (V) and about 120 V. The capacitor C can be, for example, a high voltage capacitor and/or any other suitable capacitor. In some implementations, for example, the capacitor C can have a capacitance sufficient to drive a desired charge or current across the load 1501 without a significant voltage drop across the capacitor C. In some implementations, the capacitor C can have a capacitance between about 2 $\mu$F and about 10 $\mu$F.

As described above, in some implementations, the EPT 1502 can be configured to reduce, minimize, and/or otherwise at least partially control an amount of heat associated with and/or generated by the use of the EPT 1502. In many physiological applications, it is desirable to deliver constant current during the stimulation pulse, which can result in a changing voltage drop over the current source during the stimulation pulse. In some instances, heat can result from an amount of voltage drop over the current source 1594, wherein a larger amount of voltage drop results in a larger amount of heat. Said a different way, a difference between the compliance voltage and the load voltage can result in dissipated power in the form of heat.

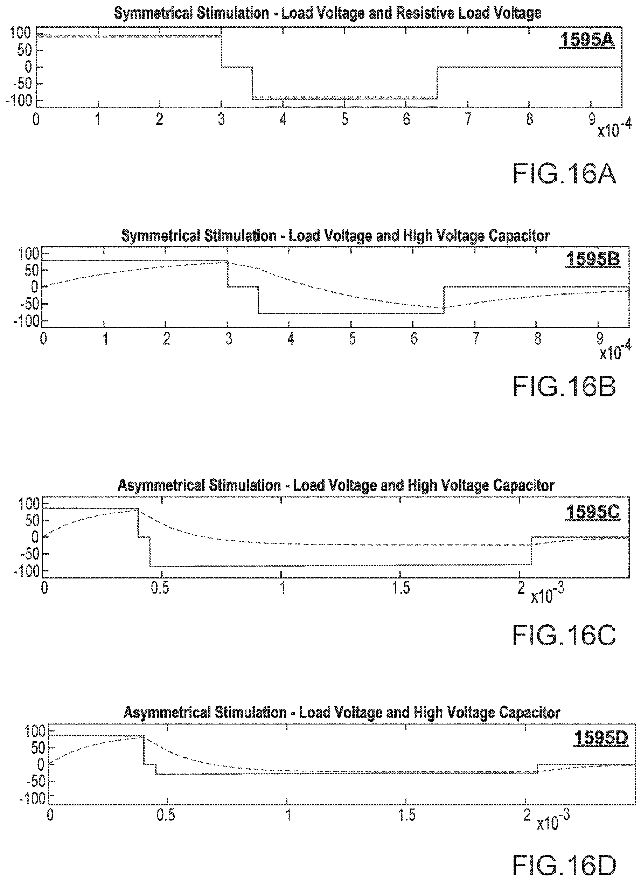

FIG. 16A is a graph 1595A that illustrates a compliance voltage (solid line) and a load voltage (dashed line) during symmetrical stimulation (e.g., where a positive phase or positive pulse of the stimulation has the same duration and the same load current as the negative phase or pulse of the stimulation but with opposite polarities). The vertical y-axis represents voltage (V) while the horizontal x-axis represents time in seconds x $10^{-4}$. The load in the example shown in FIG. 16A is a resistor and the load voltage closely follows the compliance voltage during both a positive phase and a negative phase of stimulation.

As stated above, however, the skin and tissue of a patient (e.g., the load 1501) can be modeled as a resistor in series with a capacitor. FIG. 16B is a graph 1595B that illustrates a compliance voltage (solid line) and a load voltage (dashed line) during symmetrical stimulation, when a high voltage capacitor is added to the resistor. The vertical y-axis represents voltage (V) while the horizontal x-axis represents time in seconds x $10^{-4}$. The addition of the capacitor is such that the load voltage varies significantly from the compliance voltage during the course of the stimulation pulse. During the positive phase, the load capacitor starts charging from zero, but during the negative phase, the load capacitor starts from a value that is different from zero. For example, the compliance voltage is zero at around 3.0 x $10^{-4}$ seconds to around 3.5 x $10^{-4}$ seconds, while the load voltage reaches zero at around 4.4x$10^{-4}$ seconds, as shown in FIG. 16B. During constant current stimulation, an amount of wasted energy for each pulse (which manifests as heat) is equal to an integral of a difference between the compliance voltage Vc and the load voltage Vload, multiplied by the value of the delivered current.

As a result, a difference between the load voltage and the compliance voltage is larger in the negative phase. Since the dissipated power is proportional to the difference between the compliance voltage and the load voltage, it follows that (1) a larger load capacitance will result in a larger amount of heating, (2) more heat is generated in the negative phase than the positive phase, and (3) more heat is generated in asymmetrical stimulation (e.g., where a positive phase or positive pulse of the stimulation has a different duration, voltage, and/or waveform as the negative phase or pulse of the stimulation but with opposite polarities) than in symmetric stimulation (e.g., due to the compliance voltage having the same magnitude during both positive and negative phases). For example, FIG. 16C is a graph 1595C that illustrates a compliance voltage (solid line) and a load voltage (dashed line) during asymmetrical stimulation. The vertical y-axis represents voltage (V) while the horizontal x-axis represents time in seconds x $10^{-3}$. As shown, when the positive compliance voltage is the same as the negative compliance voltage, the load voltage significantly varies from the compliance voltage during the course of the stimulation pulse.

In some instances, however, asymmetrical stimulation can be used to reduce a difference between the compliance voltage and the load voltage by, for example, varying the compliance voltage during the positive and negative phases. For example, FIG. 16D is a graph 1595D that illustrates a compliance voltage (solid line) and a load voltage (dashed line) during asymmetrical stimulation. The vertical y-axis represents voltage (V) while the horizontal x-axis represents time in seconds x $10^{-3}$. In this example, heat can be reduced during asymmetric stimulation by reducing the magnitude of the compliance voltage during the negative phase. Said another way, the reduced magnitude of the compliance voltage during the negative phase can account for, compensate for, and/or otherwise offset the reduction in the load voltage during the negative phase that is due to the load capacitor. Thus, the reduction in the difference between the variable compliance voltage and the load voltage results in less heat generation. Moreover, the reduction of the compliance voltage also reduces an amount of battery energy consumed by the EPT 1502.

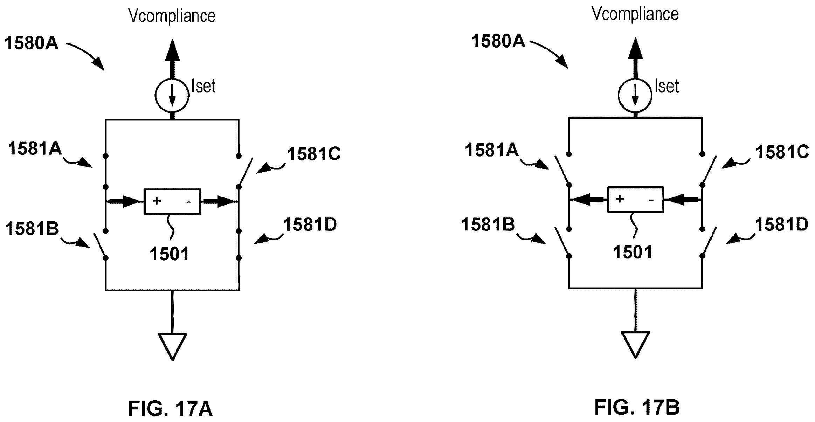

In some implementations, the EPT 1502 can be configured to transmit electrical charge to a target area of a subject and/or to an implanted device (e.g., such as any of those described herein with or without a power adapter), while limiting, reducing, and/or substantially preventing electrical charge build up and/or electrical charge imbalance in or across the load 1501 (e.g., the skin and tissue). In some implementations, the EPT 1502 can be configured to provide a constant current stimulation or energy to the target area of the subject and/or to the implanted device. For example, FIGS. 17A-17D illustrate a switch or bridge portion of the stimulating circuit 1580 included in the EPT 1502. The switch or bridge portion of the stimulating circuit 1580 (referred to herein as switch circuit 1580A), can be any suitable type of switch configured to open and/or close one or more portions of the circuit to control a flow of electric current therethrough.

For example, in the embodiment shown in FIGS. 17A-17D, the switch circuit 1580A is configured as an "H-switch" or an "H-bridge." The switch circuit 1580A can be electrically connected to the load 1501 and can be configured to transmit electrical charge thereto. More particularly, in the example shown in FIGS. 17A-17D, the switch circuit 1580A can be configured to provide and/or drive a constant current Iset through the load 1501. As shown, the switch circuit 1580A includes a first switch 1581A in series with a second switch 1581B. The switch circuit 1580A also includes a third switch 1581C in series with a fourth switch 1581D. Moreover, the first switch 1581A and the second switch 1581B are arranged in parallel with the third switch 1581C and the fourth switch 1581D. The switch circuit 1580A is arranged such that a first conductor or electrode of the load 1501 is electrically connected between the first switch 1581A and the second switch 1581B and a second conductor or electrode of the load 1501 is electrically connected between the third switch 1581C and the fourth switch 1581D. The switch circuit 1580A is posited between a voltage source (Vcompliance) and ground. In some implementations, the H-switch or H-bridge configuration can allow the switch circuit 1580A to switch between a positive phase (or positive pulse), in which an electric charge flows from Vcompliance to ground to or across the load 1501 in a first or positive direction, and a negative phase (or negative pulse), in which an electric charge flows from Vcompliance to ground to or across the load 1501 in a second or negative direction (e.g., opposite the positive direction).

FIG. 17A illustrates the switch circuit 1580A in a first configuration. As shown, the first switch 1581A and the fourth switch 1581D are in a closed configuration or state while the second switch 1581B and the third switch 1581C are in an open configuration or state, allowing current to flow from Vcompliance, across the load 1501 in a positive direction and to ground. As described above with reference to FIG. 15A, the load 1501 (e.g., skin and tissue of the patient) can be represented as a resistor in series with a capacitor. In some implementations, the load capacitor (e.g., Cload in FIG. 15A) is charged during the positive phase (or positive pulse) of stimulation. Accordingly, the first configuration can be associated with, for example, a positive phase of the stimulation.

FIG. 17B illustrates the switch circuit 1580A in a second configuration. As shown, each of the switches 1581A, 1581B, 1581C, and 1581D are in the open configuration or state. In some implementations, the second configuration can be associated with and/or can result in a ground phase, a discharge phase, a neutral phase, a zero-voltage phase, and/or the like. In some implementations, the second configuration can be an inter-phase interval, a switching interval, and/or the like associated with switching between the positive phase of the stimulation and a negative phase of the stimulation.

With each of the switches 1581A, 1581B, 1581C, and 1581D in the open configuration or state, the load 1501 is electrically isolated and/or disconnected from the switch circuit 1580A and/or the compliance voltage Vcompliance associated with the switch circuit 1580A and thus, no current is driven through the load. Moreover, because the load 1501 (e.g., the skin) does not include an ideal capacitor, the load capacitor is allowed to self-discharge at least some of the electrical charge stored by the load capacitor (e.g., built up in the skin of the subject). In some instances, the switch circuit 1580A can be configured to remain in the second configuration until a current through the load 1501 falls below a predetermined threshold. In other instances, the switch circuit 1580A can be configured to remain in the second configuration for a predetermined time. In other instances, a user can set one or more parameters associated with controlling the switch circuit 1580A (e.g., via the controller 1590 shown in FIG. 15A). As such, electrical charge built up in the load 1501 can be discharged before initiating the negative phase (or negative pulse) of the stimulation.

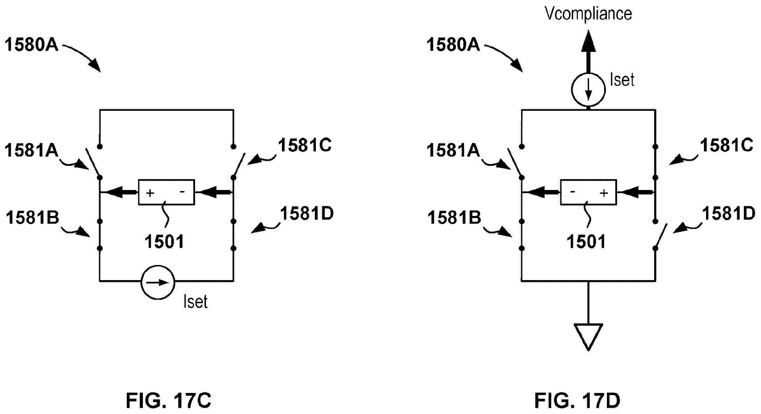

FIG. 17C illustrates the switch circuit 1580A in a third configuration. For example, in some implementations, the switch circuit 1580A can be configured to transition from the second configuration to the third configuration after a predetermined time and/or in response to a current through the load 1501 falling below a predetermined threshold. As shown, the second switch 1581B and the fourth switch 1581D are in the closed configuration or state while the first switch 1581A and the third switch 1581C are in the open configuration or state. As such, the load 1501 is electrically isolated or disconnected from a first portion of the switch circuit 1580A (e.g., the compliance voltage Vcompliance associated with the switch circuit 1580A) but electrically connected to a second portion of the switch circuit 1580A. In some instances, second portion of the switch circuit 1580A can be an optional ground or discharge portion of the switch circuit 1580A allowing the constant current to be driven through the load 1501 by the discharging of the load capacitor (e.g., to ground). More specifically, the discharging of the load capacitor (e.g., to ground) to drive the constant current through the load 1501 can result in the constant current being driven in the negative direction and/or with a negative phase. As such, the discharging of the load capacitor, for example, can initiate a negative phase of the stimulation. In some implementations, discharging the electrical charge build up in the capacitor of the load 1501 to initiate the negative phase of the stimulation can reduce an amount of energy and/or heat associated with initiating the negative phase of the stimulation, and/or the like.

FIG. 17D illustrates the switch circuit 1580A in a fourth configuration. For example, in some implementations, the switch circuit 1580A can be configured to transition from the third configuration to the fourth configuration after a predetermined time, in response to a desired amount of energy being discharged from the load capacitor, and/or in response to a current through the load 1501 falling below a predetermined threshold. As shown, the second switch 1581B and the third switch 1581C are in the closed configuration or state while the first switch 1581A and the fourth switch 1581D are in the open configuration or state. As such, the load 1501 is electrically connected to the compliance voltage Vcompliance to drive the current through the load 1501 in the negative direction (e.g., the negative phase), as shown in FIG. 17D. In some instances, the discharging of the load capacitor prior to the switch circuit 1580A transitioning to the fourth configuration can reduce and/or limit an amount of electrical charge build up in the load 1501, which can reduce and/or limit charge imbalances. In some implementations, the electrical charge build up in the load 1501 can be discharged and used to drive and/or to initiate the drive of the current in the negative direction (e.g., the negative phase) while the switching circuit 1580A is in the third configuration such that less energy is used when the load 1501 is again connected to the compliance voltage Vcompliance (FIG. 17D), thereby reducing an amount of energy and/or heat associated with initiating the negative phase, and/or the like.

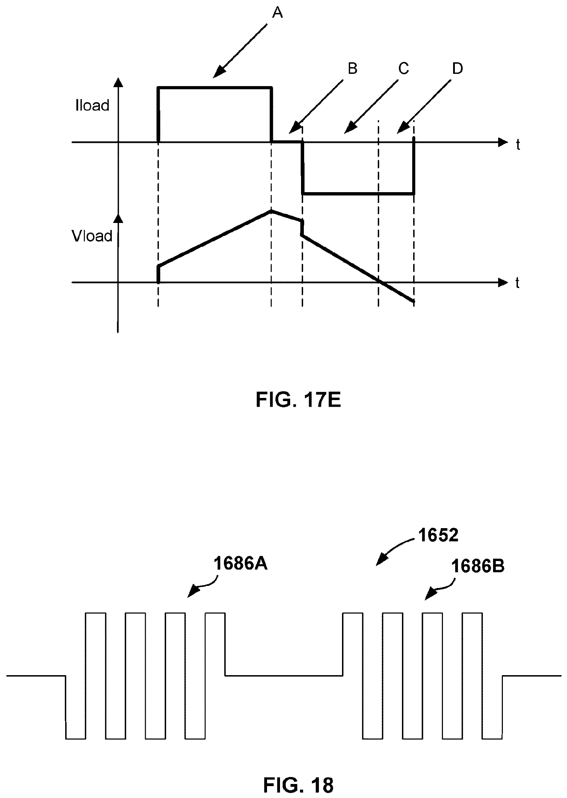

FIG. 17E is a graph illustrating an amount of current across the load 1501 (Iload) and a voltage across the load 1501 (Vload) as the switching circuit 1580A transitions through the first, second, third, and fourth configurations. For example, during the positive phase of the stimulation (indicated in FIG. 17E by the arrow A) when the switching circuit 1580A is in the first configuration (FIG. 17A), a constant current Iload having a positive phase is driven across the load 1501 and a voltage Vload across the load increases. During the inter-phase interval of the stimulation (indicated in FIG. 17E by the arrow B) when the switching circuit 1580A is in the second configuration (FIG. 17B), no current Iload is driven across the load 1501 and the voltage Vload across the load decreases in response to the load capacitor self-discharging. During an initial portion of the negative phase of the stimulation (indicated in FIG. 17E by the arrow C) when the switching circuit 1580A is in the third configuration (FIG. 17C), constant current Iload is driven across the load 1501 as a result of discharging the load capacitor (e.g., to ground). This configuration can be, for example, a negative phase - ground discharge interval. Moreover, the voltage Vload across the load decreases in response to the load capacitor discharging to ground. During a subsequent portion of the negative phase of the stimulation (indicated in FIG. 17E by the arrow D) when the switching circuit 1580A is in the fourth configuration (FIG. 17D), constant current Iload is driven across the load 1501 (e.g., in the negative direction and/or with a negative phase) by the compliance voltage Vcompliance. This configuration can be, for example, a negative phase - active discharge interval in which constant current is driven through the load 1501 (e.g., in the negative direction and/or with a negative phase) by a current source that is energized by the compliance voltage, thereby actively discharging the load capacitor. Moreover, the voltage Vload across the load continues to decrease in response to the load capacitor being discharged by the compliance voltage Vcompliance.

Although not shown in FIGS. 17A-17E, in some implementations, the switching circuit 1580A can be configured to switch back to the first configuration after the negative phase of the stimulation to initiate the positive phase of the stimulation. In some implementations, there can be a relatively long period of time (e.g., a predetermined period or predetermined time) between two successive stimulation pulses during which the switching circuit 1580A can be in an inter-phase configuration and/or the like (e.g., similar to or the same as the configuration shown in FIG. 17B). In other implementations, the switching circuit 1580A can be electrically connected to the load 1501 and a current source (Iset) can be set to zero such that no current is driven through the load 1501. In some instances, after the predetermined period or predetermined time, the switching circuit 1580A can be configured to transition or switch to the first configuration shown in FIG. 17A and the EPT 1502 can provide a positive pulse (or a stimulation pulse having the positive phase).

In some instances, the EPT 1502 can be configured to deliver bursts, pulses, and/or flows of constant current stimulation that are charge balanced and/or otherwise result in a net zero charge in the load (e.g., the skin and tissue). Said another way, the EPT 1502 can be configured to deliver energy transcutaneously through the load and to an implanted device that results in little or no electrical charge build up (e.g., a charge imbalance) in the load. In some instances, it may be desirable to correct charge imbalances at the beginning of a group or set of bursts rather than correcting charge imbalances at each individual burst, which can otherwise present challenges due to high-speed switching (e.g., about 10 $\mu$s) and time for software feedback corrections.

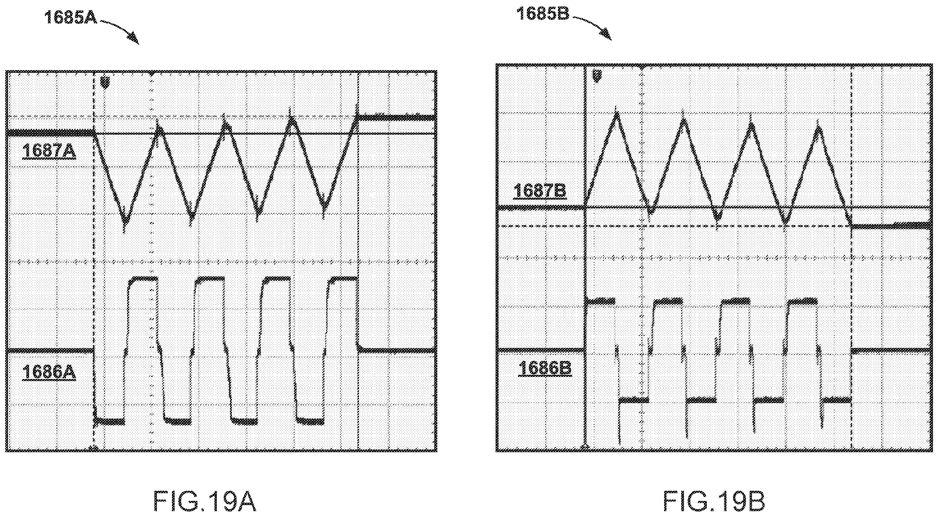

For example, FIG. 18 is a waveform 1652 illustrating two sets of bursts provided, for example, by the EPT 1502. As shown, a first set of bursts 1686A starts with a burst having a negative phase or pulse and ending with a burst having a positive phase or pulse and a second set of bursts 1686B starts with a burst having a positive phase or pulse and ending with a burst having a negative phase or pulse. While a single example of the first and the second set of bursts 1686A and 1686B, it should be understood that the EPT 1502 can provide stimulation with an alternating series of any number of the first set of bursts 1686A and the second set of bursts 1686B). FIG. 19A is a graph 1685A of an oscilloscope that shows the first set of bursts 1686A and a charge 1687A within the load associated with the first set of bursts 1686A. As shown, the first set of bursts 1686A results in an electrical charge build up in the load. Similarly, FIG. 19B is a graph 1685B of an oscilloscope that shows the second set of bursts 1686B and a charge 1687B within the load associated with the second set of bursts 1686B. As shown, the second set of bursts 1686B results in an electrical charge build up in the load that is substantially the same magnitude of the electrical charge build up associated with the first set of bursts 1686A but with an opposite polarity. Thus, the EPT 1502 can be configured to provide the second set of bursts 1686B to substantially offset a charge imbalance associated with the first set of bursts 1686A. As such, a net charge associated with the waveform 1652 is substantially zero (e.g., a net charge in or experienced by the load, the skin, the tissue, and/or portions or combinations thereof). Said another way, a residual or resultant charge associated with the first set of bursts of the waveform 1652 is substantially canceled or offset by an opposite residual or resultant charge associated with the second set of bursts of the waveform 1652.

In some instances, the EPT 1502 can be configured to provide bursts, pulses, current, and/or energy to an implanted device that includes a power adapter (such as any of those described herein) coupled to an implant (such as any of those described herein). In such instances, the power adapter can include a rectifier configured to rectify the energy received from the EPT 1502 (e.g., having the waveform 1652) into energy having any suitable and/or desired waveform. As such, the opposite polarities of the first set of bursts 1686A and the second set of bursts 1686B does not affect the rectified energy delivered from the power adapter to the implant.

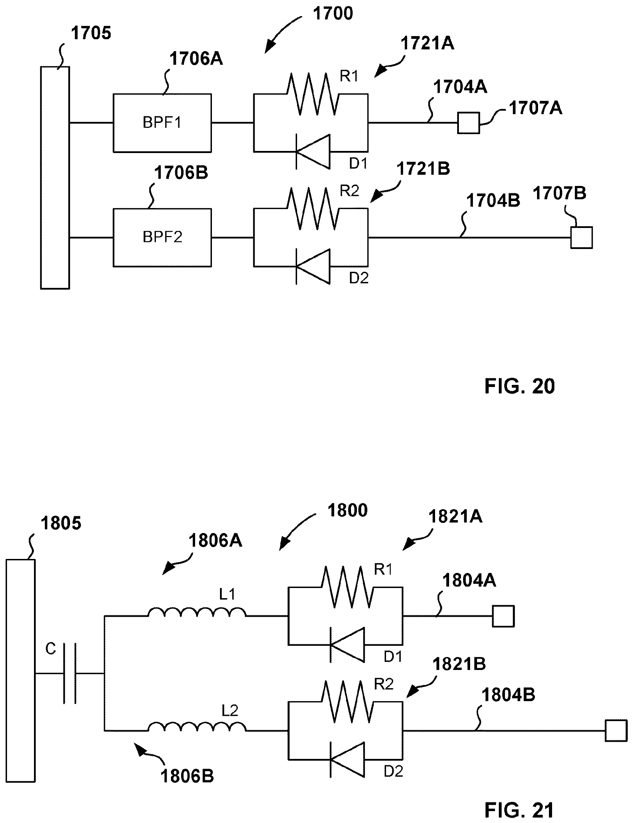

While embodiments and/or methods described herein generally include implanted devices configured to provide stimulation to a portion of the body with a single profile, in other embodiments, any of the power adapters, implants, and/or combinations thereof can be configured to provide stimulation to one or more portions of the body having any number of profiles, levels, characteristics, forms, etc. For example, FIG. 20 is a schematic block diagram of a power adapter 1700 coupled to a first implant 1704A and a second implant 1704B. As described in detail above, in some implementations the power adapter 1700 can be configured to rectify an energy transcutaneously received from a transmitter (e.g., the EPT 1502 and/or the like). The power adapter 1700 and the implants 1704A and 1704B can be similar in at least form and/or function to any of the power adapters and implants, respectively, described in detail herein.

As shown in FIG. 20, the power adapter 1700 can include a receiving electrode 1705, a first band pass filter (BPF1) 1706A, a second band pass filter (BPF2) 1706B, a first circuit 1721A, and a second circuit 1721B. More specifically, the first band pass filter 1706A is electrically coupled in series between the receiving electrode 1705 and the first circuit 1721A, which in turn, is electrically coupled in series to the first implant 1704A. Similarly, the second band pass filter 1706B is electrically coupled in series between the receiving electrode 1705 and the second circuit 1721B, which in turn, is electrically coupled in series to the second implant 1704B. Each of the first circuit 1721A and the second circuit 1721B can be a rectifying circuit configured to rectify energy (e.g., half-wave or full-wave rectification) into a desired form, level, profile, waveform, etc. For example, the first circuit 1721A can include a resistor R1 electrically coupled in parallel to a diode D1 and the second circuit 1721B can include a resistor R1 electrically coupled in parallel to a diode D2. In some embodiments, the first circuit 1721A and the second circuit 1721B can be substantially similar to any of the circuits described herein (e.g., the circuits described above with reference to FIGS. 8A-8C, 9A-9B, 10A-10B, and/or the like).

The band pass filters 1706A and 1706B can be any suitable device configured to filter or selectively control a flow of energy therethrough. For example, in some implementations, the first band pass filter 1706A can be a filter centered around a first frequency while the second band pass filter 1706B can be a filter centered around a second frequency different from the first frequency. In this manner, bursts of energy received from the transmitter (e.g., the EPT 1502) by the receiving electrode 1705 that have a carrier frequency substantially equal to or within a range of the first frequency will pass through the first band pass filter 1706A, while a carrier frequency that is not substantially equal to or not within a range of the first frequency (e.g., the second frequency) will be blocked by the first band pass filter 1706A. Similarly, bursts of energy received from the transmitter by the receiving electrode 1705 that have a carrier frequency substantially equal to or within a range of the second frequency will pass through the second band pass filter 1706B, while a carrier frequency that is not substantially equal to or not within a range of the second frequency (e.g., the first frequency) will be blocked by the second band pass filter 1706B.

As such, the first band pass filter 1706A can be configured to deliver bursts of energy having the first frequency to the first circuit 1721A, which in turn, can rectify the energy and can deliver a first rectified energy to the first implant 1704A. As shown in FIG. 20, the first implant 1704A includes a stimulating electrode 1707A configured to provide a portion of the body with stimulation having the first rectified energy. Moreover, the first band pass filter 1706A can filter out and/or not provide bursts of energy having frequencies outside a range of the first frequency. For example, the first band pass filter 1706A can be configured to filter out and/or not provide bursts of energy having the second frequency to the first circuit 1721A.

Similarly, the second band pass filter 1706B can be configured to deliver bursts of energy having the second frequency to the second circuit 1721A, which in turn, can rectify the energy and can deliver a second rectified energy to the second implant 1704B. The second implant 1704B includes a stimulating electrode 1707B configured to provide a portion of the body with stimulation having the second rectified energy. Moreover, the second band pass filter 1706B can filter out and/or not provide bursts of energy having frequencies outside a range of the second frequency. For example, the second band pass filter 1706B can be configured to filter out and/or not provide bursts of energy having the first frequency to the second circuit 1721B. In this manner, the band pass filters 1706A and 1706B can be used to separate energy received by the receiving electrode 1705 and the circuits 1721A and 1721B can be used to rectify the separate energies into two separate rectified energies having any suitable level, profile, character, waveform, etc. As such, the power adaptor 1700 can be used to provide selective stimulation to separate regions within a subject. Moreover, if stimulation is desired at electrode 1707A but not at electrode 1707B, energy having the first frequency can be provided to the receiving electrode 1705, and if stimulation is desired at electrode 1707B but not at electrode 1707A, energy having the second frequency can be provided to the receiving electrode 1705.

FIG. 21 is a schematic block diagram of power adapter 1800 coupled to a first implant 1804A and a second implant 1804B. The power adapter 1800, the first implant 1804A, and the second implant 1804B can be substantially similar to the power adapter 1700, first implant 1704A, and second implant 1704B described above with reference to FIG. 20. The power adapter 1800 includes a receiving electrode 1805, a first band pass filter 1806A, a second band pass filter 1806B, a first circuit 1821A, and a second circuit 1821B, as described above. In the example shown in FIG. 21, the power adapter 1800 includes a capacitor C, a first inductor L1, and a second inductor L2. The capacitor C and the first inductor L1 collectively form the first band pass filter 1806A and the capacitor C and the second inductor L2 collectively form the second band pass filter 1806B. In some instances, the band pass filters 1806A and 1806B can be filters centered on a frequency expressed by Equation 1 below:

$$f = \frac{1}{2\pi\sqrt{LC}} \qquad\qquad \text{Equation 1}$$

where $f$ is frequency, L is inductance, and C is capacitance.

In the example shown in FIG. 21, the capacitance of the capacitor C is the same for the first band pass filter 1806A and the second band pass filter 1806B. Thus, the band pass filters 1806A and 1806B can be configured to filter energy at different frequencies by including an inductor L1 with a first amount of inductance and an inductor L2 with a second amount of inductance different from the first amount of inductance. In this manner, the power adapter 1800 can provide energy to the first implant 1804A having a first profile, level, waveform, etc. while preventing the first implant 1804A from receiving energy with other frequencies, and energy to the second implant 1804B having a second profile, level, waveform, etc. while preventing the second implant 1804B from receiving energy with other frequencies, as described in detail above with reference to the power adapter 1700 and implants 1704A and 1704B shown in FIG. 20.

Detailed embodiments of the present disclosure have been disclosed herein or purposes of describing and illustrating claimed structures and methods that can be embodied in various forms and are not intended to be exhaustive in any way or limited to the disclosed embodiments. Many modifications and variations will be apparent without departing from the scope of the disclosed embodiments. The terminology used herein was chosen to best explain the principles of the one or more embodiments, practical applications, or technical improvements over current technologies, or to enable understanding of the embodiments disclosed herein. As described, details of well-known features and techniques can be omitted to avoid unnecessarily obscuring the embodiments of the present disclosure.

References in the specification to "one embodiment," "an embodiment," "an example embodiment," or the like, indicate that the embodiment described can include one or more particular features, structures, or characteristics, but it shall be understood that such particular features, structures, or characteristics may or may not be common to each and every disclosed embodiment disclosed herein. Moreover, such phrases do not necessarily refer to any one particular embodiment per se. As such, when one or more particular features, structures, or characteristics is described in connection with an embodiment, it is submitted that it is within the knowledge of those skilled in the art to affect such one or more features, structures, or characteristics in connection with other embodiments, where applicable, whether or not explicitly described.

Parameters, dimensions, materials, and configurations described herein are meant to be examples and that the actual parameters, dimensions, materials, and/or configurations will depend upon the specific application or applications for which the inventive teachings is/are used. It is, therefore, to be understood that the foregoing embodiments are presented by way of example only and that, within the scope of the appended claims and equivalents thereto; and that embodiments can be practiced otherwise than as specifically described and claimed. Embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the scope of the present disclosure.

As used herein, the terms "about" and/or "approximately" when used in conjunction with values and/or ranges generally refer to those values and/or ranges near to a recited value and/or range. In some instances, the terms "about" and "approximately" may mean within ± 10% of the recited value. For example, in some instances, "approximately a diameter of an instrument" may mean within ± 10% of the length of the instrument. The terms "about" and "approximately" may be used interchangeably. Similarly, the term "substantially" when used in conjunction with physical and/or geometric feature(s), structure(s), characteristic(s), relationship(s), etc. is intended to convey that the feature(s), structure(s), characteristic(s), relationship(s), etc. so defined is/are nominally the feature(s), structure(s), characteristic(s), relationship(s), etc. As one example, a first quantity that is described as being "substantially equal" to a second quantity is intended to convey that, although equality may be desirable, some variance can occur. Such variance can result from manufacturing tolerances, limitations, approximations, and/or other practical considerations.

While various embodiments have been described above, it should be understood that they have been presented by way of example only, and not limitation. Where schematics and/or embodiments described above indicate certain components arranged in certain orientations or positions, the arrangement of components may be modified. While the embodiments have been particularly shown and described, it will be understood that various changes in form and details may be made. Although various embodiments have been described as having particular features and/or combinations of components, other embodiments are possible having a combination of any features and/or components from any of embodiments described herein.

The specific configurations of the various components can also be varied. For example, the size and specific shape of the various components can be different from the embodiments shown, while still providing the functions as described herein. More specifically, the size and shape of the various components can be specifically selected for a desired or intended usage. Thus, it should be understood that the size, shape, and/or arrangement of the embodiments and/or components thereof can be adapted for a given use unless the context explicitly states otherwise.

Where methods and/or events described above indicate certain events and/or procedures occurring in certain order, the ordering of certain events and/or procedures may be modified. Additionally, certain events and/or procedures may be performed concurrently in a parallel process when possible, as well as performed sequentially as described above.

Further examples of the present invention are set out in the following numbered clauses:

1. An apparatus, comprising:

a housing configured to be coupled to an implantable electrical conductor for disposition in a body; and
a circuit at least partially disposed in the housing, the circuit configured to be electrically connected to a pick-up electrode of the implantable electrical conductor when the housing is coupled to the implantable electrical conductor,
the circuit, when the housing is coupled to the implantable electrical conductor and implanted in a body, configured to:

(i) receive, transcutaneously from an electrical pulse generating device, a first energy;
(ii) convert the first energy to a second energy; and
(iii) transmit, to the pick-up electrode, the second energy such that the implantable electrical conductor can apply, via a stimulating electrode, the second energy to a region in the body.

2. The apparatus of numbered clause 1, wherein the first energy is at a first frequency and the second energy is at a second frequency different from the first frequency.

3. The apparatus of numbered clause 1, wherein the first energy includes a first waveform and the second energy includes a second waveform different from the first waveform.

4. The apparatus of numbered clause 1, wherein the first energy is at a first frequency and the second energy is at a second frequency, the pick-up electrode is configured to receive, transcutaneously, a third energy at substantially the second frequency when the implantable electrical conductor is implanted in the body and not coupled to the housing.

5. The apparatus of numbered clause 1, wherein the first energy is at a frequency between about 30 kHz and about 100 kHz.

6. The apparatus of numbered clause 1, wherein the first energy includes alternating current and the second energy includes pulsating direct current.

7. The apparatus of numbered clause 1, wherein the first energy includes alternating current and the second energy includes a plurality of bursts of energy.

8. The apparatus of numbered clause 1, wherein the first energy is at a high frequency and the second energy includes a plurality of bursts of energy, the plurality of bursts of energy including a combination of low frequency energy bursts and high frequency energy bursts.

9. The apparatus of numbered clause 1, wherein the first energy includes a plurality of bursts of energy, the plurality of bursts of energy including a combination of low frequency energy bursts and high frequency energy bursts.

10. The apparatus of numbered clause 1, wherein the region of the body is a first region of the body, the first energy includes a plurality of bursts of energy, the plurality of bursts of energy including a combination of low frequency energy bursts and high frequency energy bursts,

the circuit configured to (1) receive the high frequency energy bursts, (2) convert the high frequency energy bursts to the second energy, and (3) transmit, to the pick-up electrode, the second energy such that the implantable electrical conductor can apply, via the stimulating electrode, the second energy to the first region in the body, the low frequency energy bursts configured to result in a local response within a second region of the body different from the first region of the body.

11. The apparatus of numbered clause 1, wherein the circuit includes a rectification circuit.

12. The apparatus of numbered clause 1, wherein the circuit includes a direct current (DC) blocking capacitor, a rectifying diode, a resistor, and a receiving electrode.

13. The apparatus of numbered clause 1, wherein the circuit includes a receiving electrode, and the circuit, when the housing is coupled to the implantable electrical conductor and implanted in a body, is configured to electrically connect to the stimulating electrode.

14. The apparatus of numbered clause 1, wherein the housing is configured to insulate the circuit from the body when the housing is coupled to the implantable electrical conductor and implanted in the body.

15. The apparatus of numbered clause 1, wherein a material of the pick-up electrode includes one of titanium or titanium-nitride.

16. The apparatus of numbered clause 1, wherein the circuit includes a rectifying diode oriented such that cathodic stimulation is provided via the stimulating electrode.

17. The apparatus of numbered clause 1, wherein the circuit incudes a rectifying diode and a capacitor in parallel with the rectifying diode, the capacitor configured to effectively short the rectifying diode at about 64 MHz.

18. The apparatus of numbered clause 1, wherein the circuit incudes a rectifying diode and a capacitor in parallel with the rectifying diode, the capacitor configured to effectively short the rectifying diode at about 128 MHz.

19. The apparatus of numbered clause 1, wherein the circuit incudes a rectifying diode, a capacitor in parallel with the rectifying diode, and an inductor in series with the rectifying diode.

20. The apparatus of numbered clause 1, wherein the circuit includes a Zener diode for electrostatic discharge (ESD) protection.

21. An apparatus, comprising:

a power adapter having a housing and a circuit at least partially disposed in the housing, the housing configured to be coupled to an implantable device for disposition in a body,
the circuit configured to be electrically connected to the implantable device when the housing is coupled to the implantable electrical conductor,
the circuit, when the housing is coupled to the implantable electrical conductor and implanted in a body, configured to:

(i) receive, transcutaneously from a power supply, first energy having a first set of characteristics;
(ii) convert the first energy to a second energy having a second set of characteristics different from the first set of characteristics; and
(iii) transfer, to the implantable device, the second energy such that the second energy powers the implantable device.

22. The apparatus of numbered clause 21, wherein the circuit includes a rectification circuit.

23. The apparatus of numbered clause 21, wherein the circuit includes a direct current (DC) blocking capacitor, a rectifying diode, a resistor, and a receiving electrode.

24. The apparatus of numbered clause 21, wherein the first energy includes alternating current and the second energy includes pulsating direct current.

25. The apparatus of numbered clause 21, wherein the first energy has a frequency of between about 30 kHz and about 100 kHz.

26. The apparatus of numbered clause 21, wherein the circuit includes a Zener diode for electrostatic discharge (ESD) protection.

27. A method, comprising:

receiving, transcutaneously and from an electrical pulse generator, first energy having at least a first characteristic;
converting, via a rectification circuit, the first energy to a second energy having at least a second characteristic different from the first characteristic; and
transferring, from the rectification circuit, the second energy to a stimulating electrode of an implantable electrical conductor such that the implantable electrical conductor applies, via the stimulating electrode, the second energy to a target nerve internal to a body.

28. The method of numbered clause 27, wherein the first characteristic is a first frequency and the second characteristic is a second frequency different from the first frequency.

29. The method of numbered clause 27, wherein the first characteristic is a first waveform and the second characteristic is a second waveform, the converting the first energy to the second energy includes halfwave rectification of the first waveform to generate the second energy having the second waveform.

30. The method of numbered clause 27, wherein the first characteristic is a first waveform and the second characteristic is a second waveform, the converting the first energy to the second energy includes fullwave rectification of the first waveform to generate the second energy having the second waveform.

**Claims**

1. An implantable power adapter (700) for use with an implantable electrical conductor (704), the implantable power adapter comprising:

a housing (710) having a conductive exterior surface;
a circuit (720) disposed within the housing, the circuit configured to be electrically connected to a pick-up electrode (705) of the implantable electrical conductor; and
an insulator (703A/703B) disposed about at least a portion of the housing, the insulator configured to electrically insulate the pick-up electrode of the implantable electrical conductor from energy external to the housing, wherein, when the implantable power adapter is coupled to the implantable electrical conductor and implanted in a body, the circuit is configured to (i) convert a first electrical energy, received from a transmitter external to the body via the conductive exterior surface of the housing, to a second electrical energy having at least one characteristic different from the first energy, and (ii) transmit the second energy to the pick-up electrode for application to a stimulating electrode of the implantable electrical conductor.

2. The implantable power adapter (700) of claim 1, wherein the circuit (720) comprises a Zener diode configured for electrostatic discharge (ESD) protection.

3. The implantable power adapter (700) of claim 1, wherein the circuit (720) comprises a capacitor in parallel with a rectifying diode, the capacitor configured to short the rectifying diode at a frequency of about 64 MHz, about 128 MHz, or both, thereby preventing nerve stimulation during MRI procedures.

4. The implantable power adapter (700) of claim 1, wherein the first electrical energy comprises alternating current at a frequency between about 30 kHz and about 100 kHz.

5. The implantable power adapter (700) of claim 4, wherein the second electrical energy comprises pulsating direct current.

6. The implantable power adapter (700) of claim 1, wherein the first electrical energy comprises a plurality of bursts of electrical energy, the plurality of bursts comprising a combination of low frequency electrical energy bursts and high frequency electrical energy bursts.

7. The implantable power adapter (700) of claim 1, wherein the insulator (703A/703B) comprises at least one of silicone, polyurethane, or polyetheretherketone (PEEK).

8. The implantable power adapter (700) of claim 1, wherein the insulator (703A/703B) is a sleeve disposed about at least the portion of the housing.

9. The implantable power adapter (700) of claim 1, wherein the conductive exterior surface of the housing (710) comprises titanium or titanium-nitride.

10. The implantable power adapter (700) of claim 1, wherein at least one of the housing (710) or the insulator (703A/703B) is configured to hermetically seal the circuit (720) from the surrounding tissue when implanted.

11. The implantable power adapter (700) of claim 1, wherein the housing (710) is configured to be retrofitted to an existing implantable electrical conductor already disposed in a body, the retrofitting comprising coupling the implantable power adapter to the implantable electrical conductor (704) by one of press-fit, crimping, or other mechanical coupling, the circuit (720) configured to automatically detect the coupling of the implantable power adapter to the implantable electrical conductor.

12. The implantable power adapter (700) of claim 1, wherein the circuit (720) is configured to be integrated into the implantable electrical conductor (704).

13. The implantable power adapter (700) of claim 1, wherein the circuit (720) is configured to convert the first electrical energy having a first waveform to the second electrical energy having a second waveform different from the first waveform.

14. The implantable power adapter (700) of claim 1, wherein the circuit (720) is configured to convert the first electrical energy having a first frequency to the second electrical energy having a second frequency different from the first frequency.

15. The implantable power adapter (700) of claim 1, wherein the circuit (720) is configured to maintain charge balance between the pick-up electrode (705) and the stimulating electrode.

FIG. 1A

SKIN/PARTITION S
ENVIRONMENT 101

TRANSMITTER
102

IMPLANT
104

ELECTRODE
19b

ELECTRODE
19a

18

FIG. 1B

30

**200**

HOUSING **210**

ELECTRODE **223a**

CIRCUIT **220**

RECTIFICATION CIRCUIT **221**

RESISTOR **222**

DIODE **224**

CAPACITOR **226**

ELECTRODE **223b**

FIG. 2

Patient
Programmer
(Mobile App)

Transmitter

302

Gel electrode

skin

301

Transcutaneous
Electrical
High Frequency
Power

303

300

Add-on
Receiver

310

Lead

304

Stimulating
Electrode

FIG. 3

**401**

RECEIVE ENERGY TRANSCUTANEOUSLY
**42**

CONVERT RECEIVED ENERGY
**44**

TRANSFER CONVERTED ENERGY
**46**

# FIG. 4

FIG. 5A

FIG. 5B

52c

FIG. 5C

52d

FIG. 5D

52e

FIG. 5E

EP 4 659 796 A2

FIG. 5F

69a 604 69b

FIG. 6A

600 610

FIG. 6B

600 610 604 69b

FIG. 6C

EP 4 659 796 A2

37

FIG. 6D

604

69a

FIG. 6E

604

610

600

FIG. 6F

604

610

5

4

600

3

2

1 620

FIG. 7A

FIG. 7B

821A

R

C

D

**FIG. 8A**

821B

ESD protection circuit

R

C

D

**FIG. 8B**

821C

ESD protection circuit

R

C

D

**FIG. 8C**

921A

FIG. 9A

921B

FIG. 9B

**1021A**

FIG. 10A

**1021B**

FIG. 10B

1102a

**FIG. 11A**

1102b

**FIG. 11B**

1102c

**FIG. 11C**

FIG.12A

FIG.12B

FIG.12C

FIG.12D

FIG.13

1405a

Tunneling Needle Stylet    Stimulation Probes    Tunneling Needle

Introducer Set

Lead Adapter

Lead Stimulation
Electrodes and Anchor

StimRouter Lead in Loader

FIG. 14A

1405b

Pocketing Needles    Pulse Generator and Receiver Pusher

Pocketing
Stylet

Silicone Sleeve Deployment Tool    Pulse Generator and
Receiver Attachment Tool

FIG. 14B

**FIG. 15A**

**FIG. 15B**

Symmetrical Stimulation - Load Voltage and Resistive Load Voltage

1595A

FIG.16A

Symmetrical Stimulation - Load Voltage and High Voltage Capacitor

1595B

FIG.16B

Asymmetrical Stimulation - Load Voltage and High Voltage Capacitor

1595C

FIG.16C

Asymmetrical Stimulation - Load Voltage and High Voltage Capacitor

1595D

FIG.16D

FIG. 17B

FIG. 17A

**FIG. 17C**

**FIG. 17D**

FIG. 17E

FIG. 18

1685B

1687B

1636B

FIG.19B

1685A

1687A

1686A

FIG.19A

FIG. 20

FIG. 21

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 50462319
- US 62976698